# EUROPEAN PATENT SPECIFICATION

(11) **EP 4 045 494 B1**
(45) Date of publication and mention of the grant of the patent: **11.10.2023**
(21) Application number: 20792606.4
(22) Date of filing: 13.10.2020
(51) Int. Cl.: C07D 401/14

(54) **SYNTHESIS OF 6-METHYL-N1-(4-(PYRIDIN-3-YL)PYRIMIDIN-2-YL)BENZENE-1,3-DIAMINE**
SYNTHESE VON 6-METHYL-N1-(4-(PYRIDIN-3-YL)PYRIMIDIN-2-YL)BENZOL-1,3-DIAMIN
SYNTHÈSE DE 6-MÉTHYL-N1-(4-(PYRIDIN-3-YL)PYRIMIDIN-2-YL)BENZÈNE-1,3-DIAMINE

(30) Priority: 14.10.2019 SG 10201909596R
(43) Date of publication of application: 24.08.2022
(73) Proprietor: Esco Aster Pte. Ltd., Singapore 139950 (SG)
(72) Inventor: ABRAHAM RAJKUMAR, Gurubatham, Singapore 486777 (SG); LIN, Xiangliang, Singapore 486777 (SG)
(74) Representative: Mewburn Ellis LLP
(86) International application number: PCT/EP2020/078761
(87) International publication number: WO 2021/074138

(56) References cited:
- WO-A1-2004/005281
- WO-A1-2013/035102
- WO-A2-2015/087343
- CN-A- 105 801 559
- CN-C- 100 537 563
- US-A1- 2006 173 182
- US-A1- 2018 334 449
- BAMBOROUGH ET AL: "N-4-Pyrimidinyl-1H-indazol-4-amine inhibitors of Lck: Indazoles as phenol isosteres with improved pharmacokinetics", BIORGANIC & MEDICINAL CHEMISTRY LETTERS, ELSEVIER, AMSTERDAM , NL, vol. 17, no. 15, 2007, pages 4363-4368, XP022144705, ISSN: 0960-894X, DOI: 10.1016/J.BMCL.2007.04.029

## Description

### Field of the Invention

The present invention relates to methods for the synthesis of compounds of Formula IIIa via compounds of Formula la, wherein R¹ is H or a nitrogen protecting group. Their use as intermediates in the synthesis of nilotinib and imatinib is also disclosed.

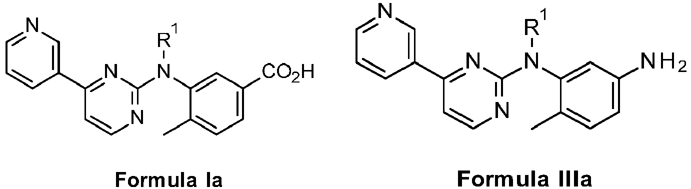

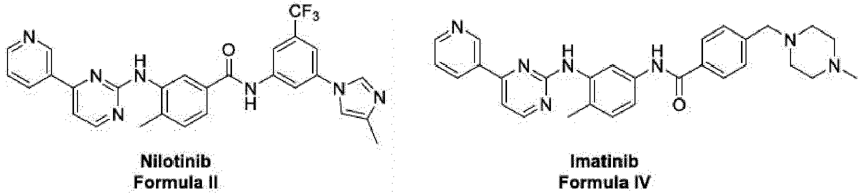

### Background

Imatinib (Formula IV) is chemically known as N-(4-methyl-3-((4-(pyridin-3-yl)pyrimidin-2-yl)amino)phenyl)-4-((4-methylpiperazin-1-yl)methyl)benzamide. Nilotinib (Formula II) is chemically known as N-(4-methyl-3-((4-(pyridin-3-yl)pyrimidin-2-yl)amino)phenyl)-4-((4-methylpiperazin-1-yl)methyl)benzamide. They are first and second generation Bcr-Abl tyrosine kinase inhibitors.

Imatinib mesylate, sold under the trade name Gleevec^{®}, is used for chronic myelogenous leukemia (CML) and acute lymphocytic leukemia (ALL) that are Philadelphia chromosome-positive (Ph+), certain types of gastrointestinal stromal tumors (GIST), hypereosinophilic syndrome (HES), chronic eosinophilic leukemia (CEL), systemic mastocytosis, and myelodysplastic syndrome. Nilotinib, in the form of the hydrochloride monohydrate salt, is sold under the trade name of Tasigna^{®} and is approved for the treatment of imatinib-resistant chronic myelogenous leukemia. Nilotinib and imatinib share a core structure (left hand side of molecule as drawn) but with reverse amide connectivity and different right hand side structure.

WO03/066613 describes two different strategies for the synthesis of imatinib. The first strategy used a C-N bond formation between two intermediates using palladium mediated Buchwald coupling conditions in the final step (Scheme 1). However, this strategy did not result in high yields of imatinib and there were other undesired by-products observed in the Buchwald coupling step which then required column purification. Expensive ligands were required to improve the yield of imatinib.

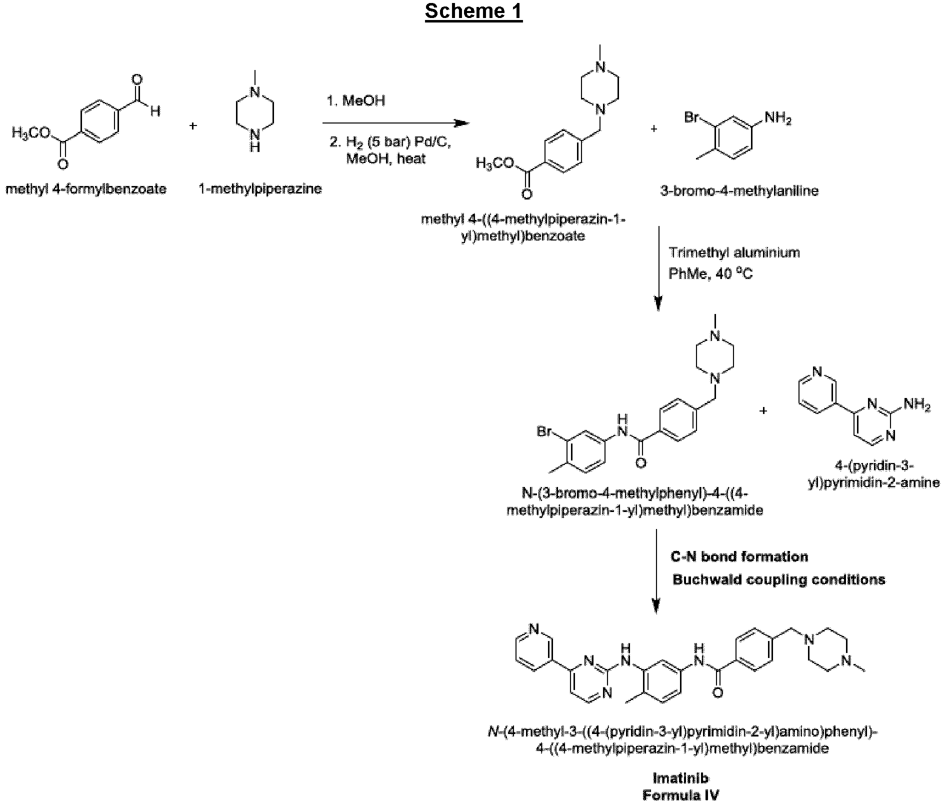

In the second strategy, pyrimidine ring construction was carried out by condensation of advanced intermediate *N*-(3-guanidino-4-methylphenyl)-4-((4-methylpiperazin-1-yl)methyl)benzamide with 3-(dimethylamino)-1-(pyridin-3-yl)prop-2-en-1-one as the final step to synthesise imatinib (Scheme 2). This strategy of pyrimidine cyclization required very high temperatures which proved to be difficult for the large-scale synthesis of imatinib.

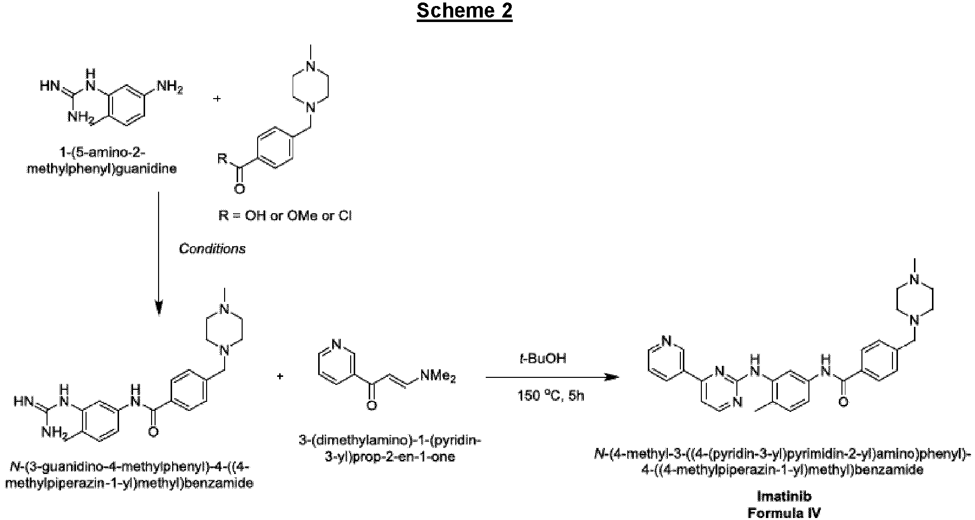

These drawbacks brought attention to the strategy of synthesising Formula III and subsequent amide bond formation between Formula III with the other intermediate as its corresponding acid, acid chloride or methyl or ethyl ester (Scheme 3). Formula III is generally obtained by synthesising *N*-(2-methyl-5-nitrophenyl)-4-(pyridin-3-yl)pyrimidin-2-amine (nitropyrimidine intermediate) followed by its reduction using different conditions and catalysts. This approach leads to two difficult obstacles, i) the synthesis of the nitropyrimidine intermediate, and ii) the reduction of the nitropyrimidine intermediate.

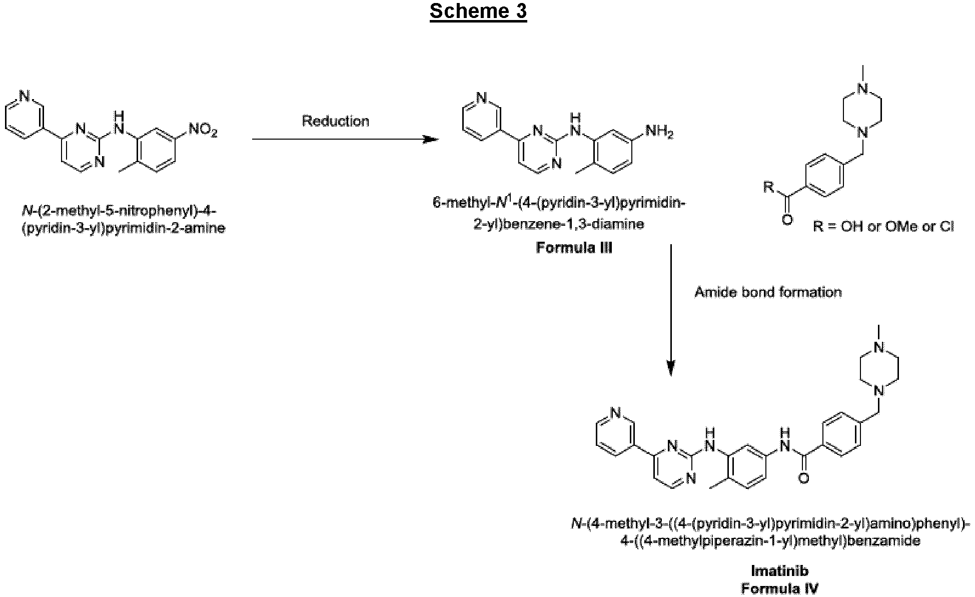

### i) Synthesis of Nitropyrimidine Intermediate

US5521184 describes the synthesis of imatinib via amide coupling of Formula III and the appropriate carboxylic acid chloride (Scheme 4). The nitropyrimidine intermediate was obtained in low yields, and was found to be contaminated with inorganic impurities. The reduction of this intermediate using palladium on carbon afforded Formula III (which is equivalent to Formula IIIa shown above where R1 is H). The main disadvantages of this process were the difficulties in the pyrimidine ring cyclization reaction and the subsequent reduction of the impure product which gave impure Formula III, leading to impure imatinib, which required column purification. Furthermore, the yields were not clearly specified and the details of amide formation of Formula III with the acid chloride were not mentioned in the patent.

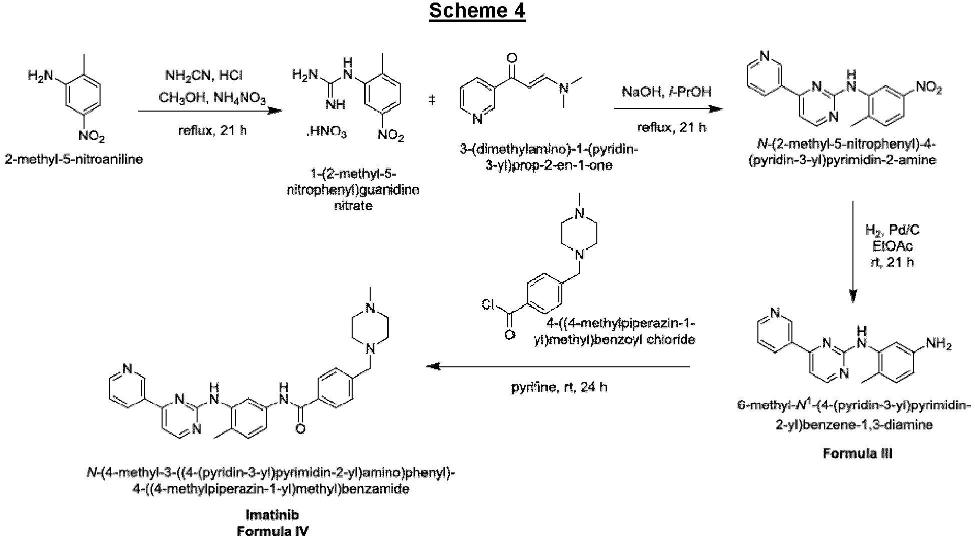

US2004248918 describes a similar strategy for the synthesis of the nitropyrimidine intermediate, which was obtained as the nitrate salt. The intermediate was claimed to be obtained in 88% yield after isolation and washing with methanol however the subsequent reduction with stannous chloride in the presence of hydrochloric acid gave the compound Formula III in only 61% yield. This could be because the yield of nitropyrimidine intermediate was not accurate, as it could have been contaminated with inorganic impurities and had to be washed with solvents after reduction. Therefore, this early process suffers from the drawbacks associated the moderate yield of Formula III and inorganic impurities formed along with the nitropyrimidine impurity. However the inventors understand that this process is the is the most followed protocol for the synthesis of *N*-(2-methyl-5-nitrophenyl)-4-(pyridin-3-yl)pyrimidin-2-amine.

US2006149160 describes the synthesis of the pyrimidine intermediate via Route A (Scheme 5). The nitropyrimidine intermediate is formed by a C-N bond formation using 2-chloro-4-(pyridin-3-yl)pyrimidine and 2-methyl-5-nitroaniline. Yield and purity were good. However the reduction step used 10% palladium on carbon with 15 volumes of ethyl acetate making it a less attractive process in terms of cost.

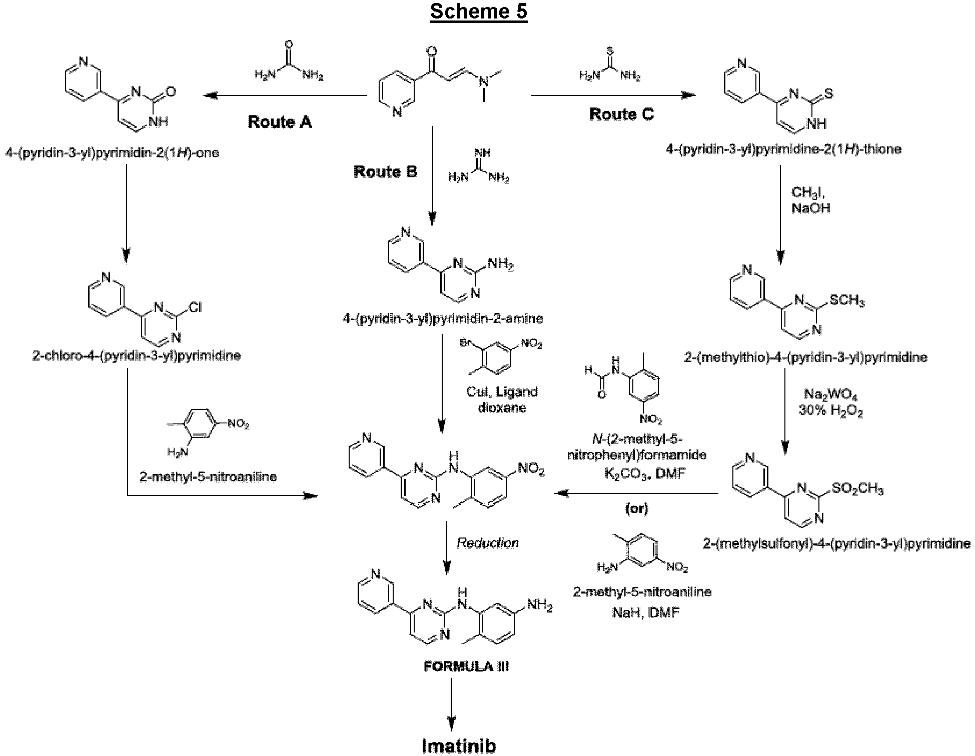

In Route B (Huaxue Yanjiu Yu Yingyong, 24(3), 484-7, 2012) the nitropyrimidine intermediate is formed by a copper mediated C-N bond formation using 4-(pyridin-3-yl)pyrimidin-2-amine and 2-bromo-1-methyl-4-nitrobenzene.

In Route C (Monatsch Chem 2010, 141, 907-911) the nitropyrimidine intermediate is formed using 2-(methylsulfonyl)-4-(pyridin-3-yl)pyrimidine and either N-(2-methyl-5- nitrophenyl)formamide or 2-methyl-5-nitroaniline (Faming Zhuanli Shenqing, 103420976,2013). The pungent smell of sulfur intermediates in this route limits its applicability on larger scales.

CN1900073A describes formation of the nitropyrimidine intermediate via an S_{N}Ar displacement between 2-chloro-4-(pyridin-3-yl)pyrimidine and 2-methyl-5-nitroaniline (Scheme 6, Route 1). The generation of pyridin-3-ylzinc(II) bromide requires anhydrous conditions which has to be standardised at larger scales.

A two-step synthesis is described in Tet. Lett. 2012, 53(49), 6657-61 which is based on a palladium-catalyzed Suzuki coupling, followed by C-N bond formation with 2-bromo-1-methyl-4-nitrobenzene catalyzed by copper(ii)oxide bound on polystyrene beads (Scheme 6, Route 2). Although these approaches provide a shorter route to *N*-(2-methyl-5-nitrophenyl)-4-(pyridin-3-yl)pyrimidin-2-amine the drawbacks associated with the reduction of this intermediate are still present.

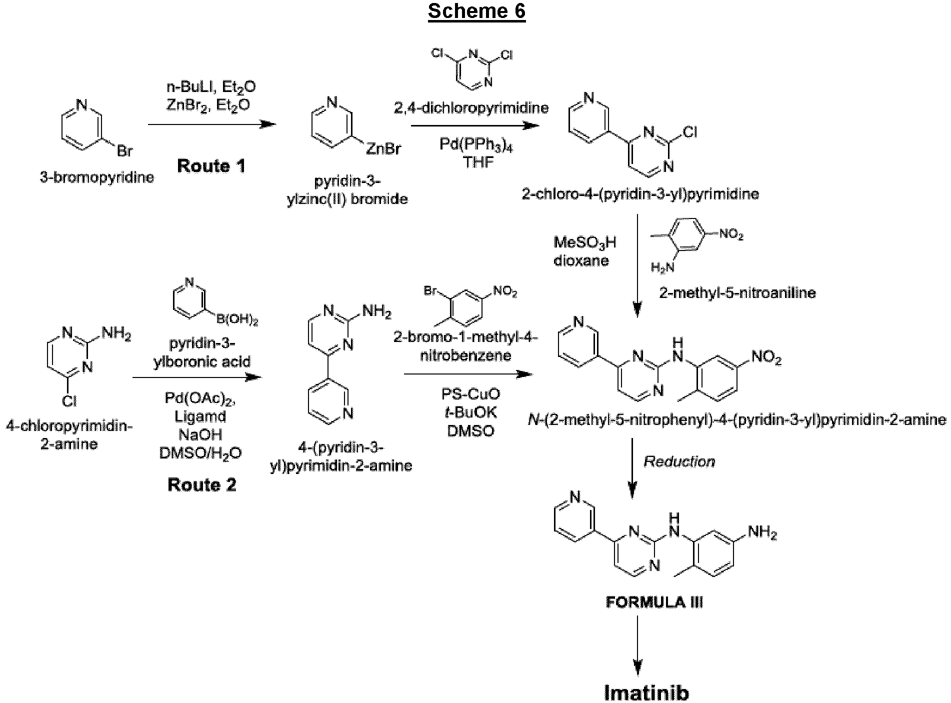

### ii) Reduction of the Nitropyrimidine Intermediate

US2006173182 uses stannous chloride for the reduction of the nitropyrimidine intermediate, *N*-(2-methyl-5-nitrophenyl)-4-(pyridin-3-yl)pyrimidin-2-amine, under acidic conditions. The crude product (Formula III) was carried forward to the final amidation step to afford imatinib, which was obtained as the trichloride monohydrate. Imatinib is marketed as the mesylate salt, necessitating treatment and recovery of the product as the free base before salt formation, adding an additional step to the process.

Variations on the stannous chloride mediated reduction of *N*-(2-methyl-5-nitrophenyl)-4-(pyridin-3-yl)pyrimidin-2-amine have been described by changing the conditions, solvents and work-up procedures.

For example, WO2004/099187 describes using 5 equiv. of stannous chloride and a mixture of ethanol and ethylacetate (1:10 v/v) as solvents and under reflux conditions. This is also described and cited in WO2008024829, Faming Zhuanli Shenqing 10122508523 2008, Bioorganic & Medicinal Chemistry,22(1), 623-632; 2014. The product was carried forward after basic work-up for the next step without purification.

Reduction with stannous chloride using protic solvents such as methanol and ethanol under reflux conditions has been described in, for example, WO2009063054 and Tet. Lett. 2012, 53(49), 6657-61.

Use of 4-5 equiv. of stannous chloride under acidic conditions with HCl as solvent is described in WO2004108699, and cited in other patent applications (KR20100027898) and in journals (Molecules 14(10), 4166-4179, 2009; Chemical Science Review and Letters, 3(11), 462-477, 16 pp.; 2014). After completion of the reaction, the mixture was neutralized with sodium hydroxide and extracted with solvents such as chloroform and ethyl acetate (as described in WO2008058037 and WO2008137794). The yields obtained by this method often range from 60-70% and serious precautions have to be taken while adding the compound to a mixture of SnCl₂ and HCl.

Using stannous chloride and then bases like K₂CO₃ for neutralization, followed by extraction with ethyl acetate resulted in variable yields (46% yield as described in US2008/300268; 80% yield as described in J.Med.Chem, 48(1), 249-255, 2005; Bioorg. Med.Chem. Lett, 20(17), 5232-6, 2010; Eur.J. Med. Chem, 46 (12), 5817-5824, 2011).

Palladium on carbon is also used in the reduction of the nitropyrimidine intermediate. Pd/C mediated reductions have been carried out at room temperature under H₂ atmosphere with different solvents such as ethyl acetate (US2004224967, US2006149061, Chem.Comm. 46(7), 1118-20, 2010, Org. Biomol. Chem 7(24), 5129-36, 2009, Angew. Chem. Int. Ed., 52 (33), 8551-56, 2013), MeOH (Angew. Chem. Int. Ed., 54(1), 179-183; 2015); THF (US5521184, J. Med.Chem. 52(8), 2265-2279, 2009, ChemMedChem 2010, 5, 130 - 139), and MeOH/THF mixture (Heterocycles, 89(3), 693-708; 2014 and WO2017073065).

Reductions using palladium on carbon in combination with *in situ* hydrogen generators such as ammonium formate (Farming Zhuanil Shanqing 102199146, 2011, Faming Zhuanli Shenqing, 104341387, 2015 and Archiv der Pharmazie 350(3-4), 2017) and sodium hypophosphite (as described in Indian Pat. Appl. IN1073/MUM/2003, Granted Number 247220) under reflux conditions have been described. However on a large scale it is difficult to control the hydrogen gas evolution, limiting the usefulness of these reactions in manufacturing protocols.

The reduction using hydrazine hydrate over 10% Pd/C, as reported in, for example, WO2011039782 is shown to give higher yields (99%) of the aminopyrimidine intermediate (Formula III). However the inventors note that the use of Pd/C on a larger scale, especially under heating conditions, poses safety issues and large scale disposal of Pd waste is a problem.

The reduction of the nitropyrimidine intermediate using Raney nickel and 80% hydrazine hydrate (added in two portions) is described in US2008194819. However this reduction process gave Formula III and by-products. These by-products are formed due to less active Raney nickel. Therefore highly active Raney nickel is required to carry out the reaction to limit the formation of these by-products. Furthermore, these by-products crystallised faster than the desired aminopyrimidine intermediate (Formula III) and proper care had to be taken to isolate the desired product without the contamination of these by-products. A maximum yield of 80% of Formula III was obtained under these conditions.

Seryya Khimichnykh Navuk, (3), 79-86; 2013 also describes similar reaction conditions and Formula III was obtained in a yield of 74-80%. Similar conditions using Raney nickel and hydrazine hydrate with different solvents such as EtOH under reflux conditions is described in Faming Zhuanli Shenqing, 103420976, 2013 and THF as solvent at room temperature is described in Faming Zhuanil Shenqing 102796110, 2012. The use of Raney Nickel under hydrogen atmosphere using THF as solvent is reported to yield 90% of Formula III as described in Zhongguo Yaoxue Zazhi, 43(3), 228-229, 2008 and reproduced in Yuaxue Huaxue, 35(11) 2377-82. Kompella Amala et al. reported reduction using Raney nickel under an atmosphere of hydrogen at room temperature in WO2013035102 and in Organic Process Research & Development 16(11), 1794-1804, 2012. In order to obtain high yields the Raney nickel required washing with purified water and long reaction times (45 hours) were required. It is also noted that 30% wt/wt Raney nickel was essential for completion of the reaction. Generally, WO2013035102 describes reduction of a nitropyrimidine intermediate to the corresponding amine (which is analogous to Formula III herein), which is then reacted to afford imatinib base.

Reduction of the nitropyrimidine intermediate using sodium sulfide or sodium polysulfide is described in WO2008/117298. In a separate publication (Monatsch Chem 2009, 140, 619-23) the reduction using sodium dithionite is described but the maximum yield that was achieved was only 81%. The toxicity and the unpleasant smell associated with sulfur containing reagents limit their use on a larger scale.

An FeCl₃-mediated reduction is described in a number of journal publications (Organic Process Research & Development 12(3), 490-495, 2008; Monatsch Chem 2010, 141, 907-911and Shenyang Yaoke Daxue Xuebao, 27 (5), 361-4, 2010). Iron oxide mediated reduction is described in Faming Zhuanil Shenqing 101701015, 2010) using hydrazine hydrate as the hydrogen source and methanol as solvent. Although iron-mediated reductions are less toxic than metals such as palladium and tin, hydrazine hydrate is still highly toxic and has to be used with FeCl₃ for improved yields of up to 80%, again limiting the usefulness of these methods on a manufacturing scale.

Reduction using iron powder under acidic conditions (acetic acid), with ethanol as solvent under reflux is described in Journal of Nuclear Medicine, 52 (8), 1301-07, 2011) or in combination with ammonium chloride. Methanol as solvent under reflux conditions is described in Faming Zhuanli Shenqing, 107652266, 2018. These methods lead to a lower yield than reduction using FeCl₃ and hydrazine hydrate.

Reduction using a combination of sodium borohydride and CoCl₂ as reducing agents afforded only 71% of Formula III (Nuclear Medicine and Biology 34 (2007) 153-163).

Zinc and ammonium chloride as reducing agents in THF afforded 80% of the product Formula III under microwave heating conditions.

Hydrazine hydrate as a reducing agent without any metal catalyst in water at reflux and in hexane has been described in Faming Zhuanil Shenqing 103242116, 2013 and Faming Zhuanil Shenqing 103508827, 2014 respectively. Though the reported yields are quantitative the purity of the product is not specified. Another reduction catalyst system which includes hydrazine hydrate and HzOz-treated activated carbon using DMF as solvent under heating conditions has been reported in Journal of Chemical Research, 41(9),509-512; 2017. However the scalability of this protocol is yet to be explored.

Nickel nanoparticle, hydrazine hydrate in ethanol under heating conditions (Russian Journal of General Chemistry, 88(3), 410-417; 2018) gave Formula III in 77% yield on a lab scale. Other methodology reported hydrazine hydrate in the presence of a nanocatalyst based on cobalt-nickel nanoparticles (Russian Journal of Organic Chemistry,54(6), 943-944; 2018) afforded Formula III in 77% yield. However the applicability of these methodologies on a larger scale is not yet clear.

A different approach to avoid the late stage reduction after pyrimidine cyclization is described in Repub korean kongkae Taeho Kongbo 2012089039 and proceeded via acylated protected Formula III (Scheme 7). Although this is a good strategy, two extra steps were required to obtain Formula III and the yields for the individual steps were not disclosed. Another approach to generate acylated Formula III was reported in Faming Zhuanil Shenqing 102796074 and used a copper mediated C-N bond formation (Scheme 7).

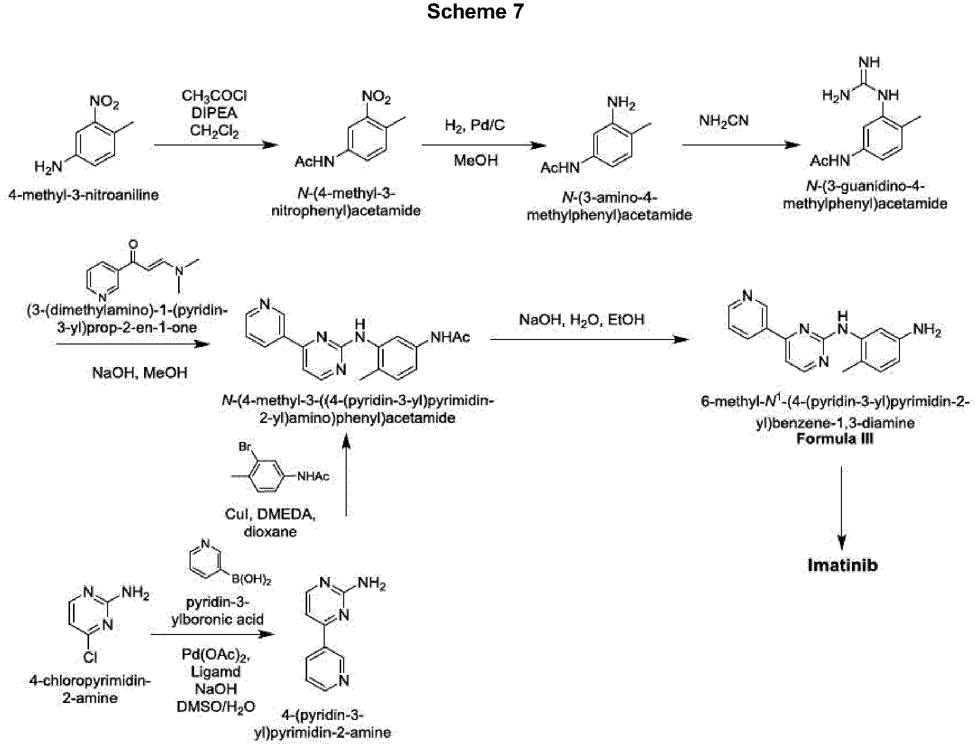

Bamborough et al. (in Bioorg. Med. Chem. Lett (2007), vol. 17, no. 15, pg. 4363-4368) describe the synthesis of 2,4-dianilino pyrimidine compounds from the corresponding carboxylic acid precursor under standard Curtius rearrangement conditions, namely (a) diphenylphosphoryl azide, EtsN, tert-butanol, reflux, and (b) HCl in dioxane, CH₂Cl₂ at room temperature.

CN 105801559 A and CN 100537563 C each disclose a method of making a compound that corresponds to Formula Villa herein, by a reaction between a halopyrimidine and an aniline.

WO 2015/087343 A2, US 20180334449 and WO 2004/005281 A1 each disclose a method of making a compound that corresponds to Formula Villa herein, by a condensation between a nitrate salt of an alkyl 3-guanidino-4-methylbenzoate and 3-(dimethylamino)-1-(pyridin-3-yl)prop-2-en-1-one. The resultant ester compound is then hydrolysed to give the corresponding carboxylic acid, which is reacted further to give useful products. Nilotinib is the product in WO 2015/087343 A2, while US 20180334449 A1 is particularly directed towards purification of nilotinib from a free base raw material precursor.

Despite considerable work in this area there remains a need in the art for efficient, safe and scalable routes to imatinib and nilotinib. The present invention has been devised in light of the above considerations.

### Summary of the Invention

The inventors have devised an efficient synthesis of imatinib and nilotinib. The route is divergent, meaning that late stage intermediate Formula I (and protected forms thereof) is common to the synthesis of both imatinib and nilotinib. Formula III is efficiently obtained from Formula I for use in the synthesis of imatinib.

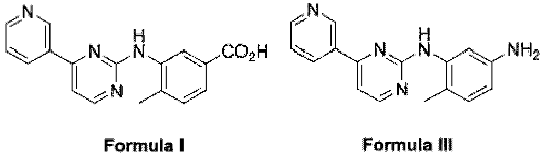

As described above, hitherto syntheses of Formula III have typically proceeded via a nitropyrimidine intermediate which is then reduced to afford the amine. As large scale reduction of the nitropyrimidine intermediate often uses toxic or odorous chemicals and requires expensive metal catalyst systems, this poses challenges for the synthesis of imatinib at manufacturing scale. Furthermore the synthesis of the nitropyrimidine intermediate often proceeds in low yield with the formation of by-products, while the use of toxic reagents late in the synthesis causes additional complications and may necessitate additional purification steps and/or checks in good manufacturing practice where imatinib is synthesized for prescription to patients. Other methods have relied on late stage C-N cross-coupling reactions, for example Buchwald or Ullmann reactions, or late generation of the pyrimidine ring, that is using pyrimidine ring construction strategy at a later stage.

In a first aspect the present invention provides a method for the synthesis of a compound of Formula IIIa: 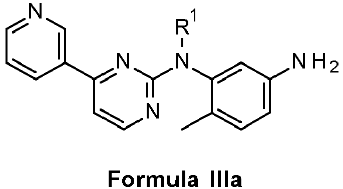
wherein R¹ is H or a nitrogen protecting group, the method comprising treating a compound of Formula la with reagents to form an acyl azide: 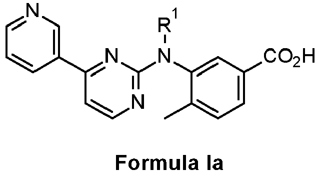
wherein R¹ is H or a nitrogen protecting group;
followed by decomposition of the acyl azide to provide an isocyanate; which undergoes nucleophilic attack with an alcohol or water. That is, the isocyanate undergoes nucleophilic attack.

That is, the carboxylic acid moiety -COOH is reacted to form -CONs which decomposes to -N=C=O.

It will be appreciated that the use of alcohol or water nucleophile determines the structure of the resultant carbamate: -NHCOOH with water, which typically undergoes COz elimination *in situ* to form the amine, or -NHCOOR⁴ with use of an alcohol R⁴OH. The amine of -NHCOOR⁴ may be unmasked with deprotection of NR¹ (wherein R¹ is a nitrogen protecting group) or in a separate step using methods known in the art. Preferably, the amine of -NHCOOR⁴ is unmasked with deprotection of NR¹ in a single step.

In some embodiments the isocyanate undergoes nucleophilic attack with an alcohol R⁴OH to provide a compound of Formula XIII: 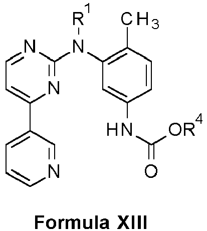 wherein R⁴ is an optionally substituted C₁₋₇ alkyl.

In some embodiments R⁴ is an optionally substituted C₁₋₄ alkyl group.

In some embodiments R⁴ is selected from t-butyl and an optionally substituted benzyl group.

In some embodiments R⁴ is selected from t-butyl, benzyl and 2,4-dimethoxybenzyl.

In some embodiments R⁴ is t-butyl.

In some embodiments, the reagents for acyl azide formation comprise diphenylphosphoryl azide (DPPA) or sodium azide.

In some embodiments, the reagents for acyl azide formation comprise diphenylphosphoryl azide (DPPA).

In some embodiments R¹ is *t*-butyloxycarbonyl.

In some embodiments R¹ is not H.

Use of a protecting group improves solubility and therefore offers operational advantages. It may also be helpful in preventing unwanted side reactions at the secondary amine. It will be recognised that the invention envisages that R¹ may change (that is, deprotection may occur) during methods claimed.

In some embodiments R¹ is not H and the method further comprises a deprotection step to remove the R¹ nitrogen protecting group.

In some embodiments the deprotection step uses trifluoroacetic acid and the deprotection affords a compound of Formula III as the free base:

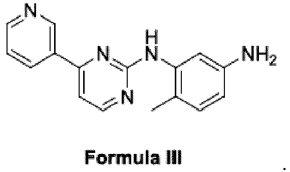

In some embodiments the deprotection step uses hydrochloric acid and the deprotection affords a compound of Formula III as the hydrochloride salt: 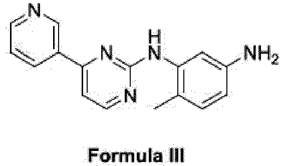 .HCl.

Disclosed herein, not forming part of the invention, is a compound of Formula la and its use in the synthesis of imatinib and nilotinib: 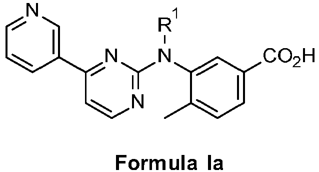 wherein R¹ is a nitrogen protecting group.

Also disclosed herein, not forming part of the invention, is a compound of Formula Villa and its use in the synthesis of imatinib and nilotinib: 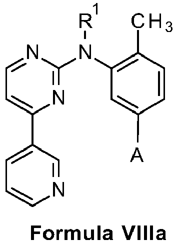 wherein R¹ is H or a nitrogen protecting group; and A is CN.

R¹ may be selected from *tert*-butyloxycarbonyl (Boc), fluorenylmethyloxycarbonyl (Fmoc), α,α-dimethyl-3,5-dimethoxybenzyloxycarbonyl (Ddz), 2-(4-biphenyl)isopropoxycarbonyl (Bpoc), *p*-methoxybenzyl (PMB) or 2,4-dimethoxybenzyl.

In some embodiments of the method of the invention, the method further comprises the step of providing the compound of Formula la by effecting a coupling reaction between a compound of Formula XI: 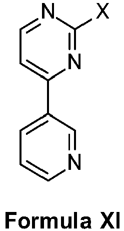
and a compound of Formula Xlla: 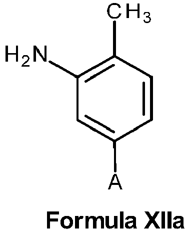
to provide a compound of Formula Villa: 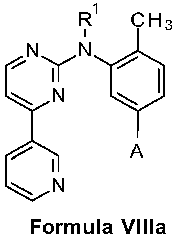
and hydrolysing the compound of Formula Villa to produce the compound of Formula la;
wherein R¹ is H;
X is a halogen or a pseudohalide;
and A is COOR² or CN;
wherein R² is an optionally substituted C₁₋₄ alkyl.

It will be appreciated that, as an alternative to a halogen, X can be a so-called pseudohalide for the purpose of the coupling reaction, for example a triflate (OTf) or a tosylate (OTs). The chemistry of a pseudohalide group resembles that of a halogen in the coupling reaction.

Thus in some embodiments, X is a halogen, OTf or OTs. In some embodiments, X is a halogen or OTf. Preferably X is a halogen. More preferably X is Cl.

In some embodiments A is COOR². Thus, in these embodiments, the method comprises effecting a coupling reaction between a compound of Formula XI: 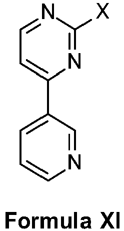
and a compound of Formula XII: 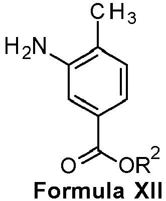
to provide a compound of Formula VIII: 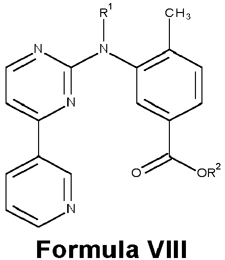
wherein R¹ is H;
R² is an optionally substituted C₁₋₄ alkyl; and
X is a halogen or a pseudohalide.

In some embodiments, X is a halogen, OTf or OTs. In some embodiments, X is a halogen or OTf. Preferably X is a halogen. More preferably X is Cl.

In some embodiments the method comprises providing a compound of Formula XI by coupling of a compound of Formula IX 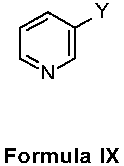
with a compound of Formula X 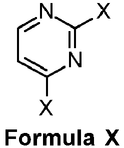
wherein each substituent X in Formula X is independently a halogen, and may also be a pseudohalide; and Y is a halogen, a pseudohalide, or other group suitable for cross coupling.

In some embodiments, each substituent X in Formula X is independently a halogen, OTf or OTs, optionally Cl.

In some embodiments, Y is a halogen, OTf, OTs, or other group suitable for cross coupling.

In some embodiments, Y is selected from a halogen, OTf, OTs, -SnR^{A}₃, -B(OH)₂ and 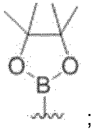 wherein R^{A} is C₁₋₄ alkyl. Preferably R^{A} is methyl or n-butyl.

In some embodiments, Y is a halogen, OTf or -B(OH)₂.

In some embodiments, Y is Cl. In some embodiments, Y is -B(OH)₂.

In some embodiments, the method comprises providing a compound of Formula XI by coupling of a compound of Formula IX with a compound of Formula X, wherein the method comprises treating the compound of Formula IX with magnesium to form a Grignard reagent. For example, the Grignard reagent may be an organomagnesium halide, such as an organomagnesium chloride or an organomagnesium bromide.

In some embodiments, the method comprises providing a compound of Formula XI by coupling of a compound of Formula IX with a compound of Formula X, wherein the method comprises treating the compound of Formula IX with zinc to form an organozinc Negishi reagent. For example, the organozinc Negishi reagent may be an organozinc halide, such as an organozinc bromide.

In some embodiments, the method comprises providing a compound of Formula XI by Suzuki coupling of a compound of Formula IX with a compound of Formula X; wherein Y is -B(OH)₂ or

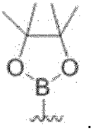

In some embodiments of the method of the invention, the method further comprises the step of providing the compound of Formula la by reacting a compound of Formula VII: 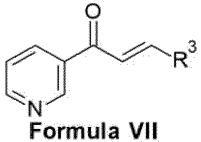
with a compound of Formula VIa: 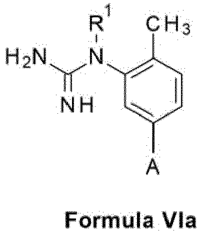
to provide a compound of Formula Villa: 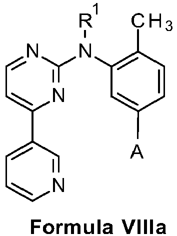
and hydrolysing the compound of Formula Villa to produce the compound of Formula Ia;
wherein Formula Vla is the free base or a chloride, bromide, iodide, mesylate, sulfate, phosphate, nitrate, acetate, oxalate, citrate, or tartrate salt; A is CN; R¹ is H or a nitrogen protecting group; and R³ is a suitable leaving group, optionally dialkyl-substituted nitrogen.

In some embodiments, R¹ is H.

In some embodiments, R³ is NMe₂.

### Detailed Description of the Invention

Aspects and embodiments of the present invention will now be discussed. Further aspects and embodiments will be apparent to those skilled in the art.

In some aspects, the present invention provides a synthesis for a compound of Formula IIIa: 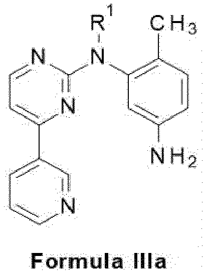 which is an intermediate in the synthesis of imatinib.

The synthesis uses an intermediate of Formula la: 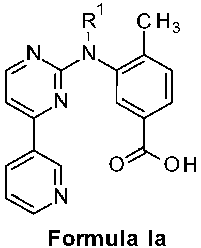 wherein the compound of Formula IIIa is obtained via a Curtius rearrangement. Formula la is also a useful intermediate for the synthesis of nilotinib. As a consequence, the present invention provides a useful divergent synthesis for two useful anti-cancer agents via a useful late intermediate. This means that intermediates of Formula la may be obtained via a single process in a single chemical plant.

The process of the invention further avoids the late-stage reduction of a nitro group typically used in the methods of the prior art for the synthesis of imatinib. As described above, the reductions of the prior art may present complications in terms of scale, where gases or reactive reagents such as Pd/C are used, and often use heavy metals such as tin, traces of which may cause toxicity concerns.

Formula la is converted into Formula IIIa via acyl azide formation and a subsequent Curtius rearrangement as shown:

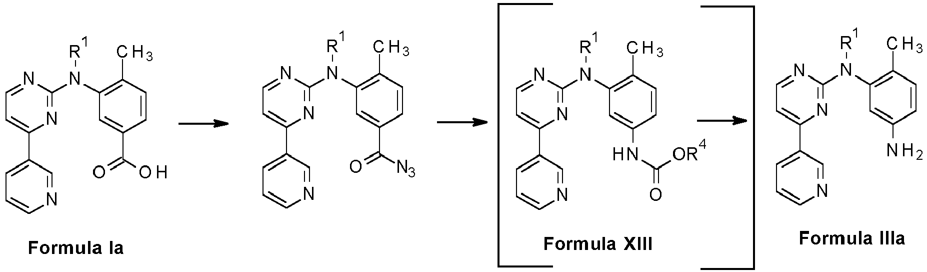

R¹ as defined herein is hydrogen or a nitrogen protecting group.

During the acyl azide formation step and subsequent Curtius rearrangement, R¹ is typically a nitrogen protecting group, suitably an acid labile protecting group, for example selected from tert-butyloxycarbonyl (Boc), α,α-dimethyl-3,5-dimethoxybenzyloxycarbonyl (Ddz), 2-(4-biphenyl)isopropoxycarbonyl (Bpoc), *p-*methoxybenzyl (PMB), and 2,4-dimethoxybenzyl. Another nitrogen protecting group is fluorenylmethyloxycarbonyl (Fmoc). Use of a protecting group improves the solubility of the compound, facilitating further reaction. Furthermore a protecting group prevents reaction of the amine in subsequent steps until needed.

Suitable reagents and conditions for acyl azide formation are known to a person skilled in the art, and may include, for example, treatment of the carboxylic acid with sodium azide in the presence of triphenylphosphine and a suitable acyl chloride forming reagent, e.g. trichloroacetonitrile or trichloroisocyanuric acid, or with DPPA optionally in the presence of a Lewis acid, for example zinc triflate, silver triflate, boron trifluoride diethyl ethereate, silver carbonate, silver oxide and zirconium halides.

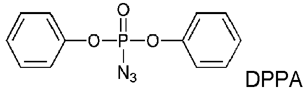

DPPA is commercially available, from, for example Merck^{®}, and is also known as phosphoric acid diphenyl ester azide.

Sodium azide can also be used in conjunction with a base and an activating agent. Suitable activating agents include cyanuric chloride and triphosgene. Suitable bases have a pKₐ > 5, for example >10, and may be, for example, selected from EtsN or N-methyl morpholine.

Azide formation can proceed via conversion of the acid to an ester using techniques known to the person skilled in the art, then reduction to the aldehyde using reagents such as DIBAL-H or borane THF. The acyl azide is formed by reaction of the aldehyde with an oxidising agent, for example tert-butyl hypochlorite, and sodium azide in a chlorinated solvent, for example chloroform or dichloromethane.

Azide formation can proceed via the acyl chloride of the acid. Suitable reagents for acyl chloride formation are oxalyl chloride or thionyl chloride, suitably in the presence of a catalyst such as DMF. Suitable solvents include dichloromethane or toluene. Following formation of the acyl chloride reaction with an azide source such as sodium azide will yield the acyl azide.

Azide formation can proceed by activation of the acid using peptide coupling reagents such as propanephosphonic acid anhydride (T3P), 1-ethyl-3-(3-dimethylaminopropyl)carbodiimide (EDC) or 2-(1*H*-benzotriazol-1-yl)-1,1,3,3-tetramethyluronium hexafluorophosphate (HBTU) with an organic base. In one embodiment T3P is used. Suitable bases may have a pKₐ > 5, for example >10, and may be, for example, selected from EtsN or DIPEA. This is followed by treatment with an azide source, for example sodium azide.

The acyl azide is heated, resulting in thermal decomposition to provide the isocyanate which undergoes nucleophilic attack.

Suitably, in methods of the present invention, the reagents for acyl azide formation are diphenylphosphoryl azide (DPPA) with a base.

Suitable bases may have a pKₐ > 5, for example >10, and may be, for example, selected from triethylamine, 1,8-diazabicyclo[5.4.0]undec-7-ene (DBU), 2,3,5-collidine, sodium carbonate, potassium carbonate, cesium carbonate and tripotassium phosphate or *N*,*N*-diisopropylethylamine (DIPEA or Hünig's Base). In some cases, the base is DIPEA.

Suitably, the reaction uses a polar aprotic solvent. Suitable polar aprotic solvents may include acetonitrile, dichloromethane and DMF. Alternatively, the reaction may use a non-polar solvent. Suitable non-polar solvents may include toluene and dioxane. In some cases, the solvent is toluene.

An alcohol or water may be used as nucleophile, and may be used as a co-solvent, particularly where alcohol is used. Use of an alcohol is preferred. Changing the alcohol changes the structure of the resulting carbamate (Formula XIII), with the R⁴ group relating to the structure of alcohol R⁴OH. The resulting carbamate may be considered an amine with an ester protecting group.

Suitable alcohols will be apparent to the skilled person and may include alcohols in which R⁴ is optionally substituted C₁₋₇ alkyl. In some embodiments, R⁴ is optionally substituted C₁₋₄ alkyl. The alkyl may be linear or branched. In some embodiments the alkyl is unsubstituted. In some embodiments the alkyl is substituted with, for example, halogen or optionally substituted aromatic groups. Optionally substituted aromatic groups may be substituted with, for example, C₁₋₄alkyl (for example methyl), OH, OC₁₋₄alkyl (for example, OMe) and halogen. For example, R⁴ may be optionally substituted benzyl. In some embodiments, R⁴OH is 4-methyl benzyl alcohol or 2,4-dimethoxy benzyl alcohol. In some embodiments, R⁴OH is 2,4-dimethoxy benzyl alcohol.

In some preferred embodiments, R⁴ is *t*-butyl such that the aryl amine generated in the Curtius rearrangement is Boc-protected. This simplifies characterization of the rearrangement product, and both Boc-protecting groups can be removed together using methods described in the art, for example by treatment with an acid, such as trifluoroacetic acid in a suitable solvent such as dichloromethane or hydrochloric acid in a suitable solvent, for example, acetone or dioxane.

By this method Formula III can be isolated as a free base or as a salt of the acid used for deprotection. In one embodiment Formula III is isolated as the hydrochloride salt following treatment with hydrochloric acid.

Formula I may be formed by hydrolysis and, where appropriate, deprotection at R¹ of a compound of Formula Villa: 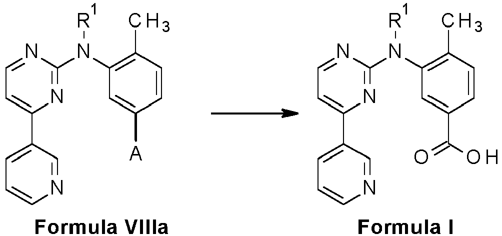 wherein A is COOR² or CN, R¹ is H or a nitrogen protecting group and R² is optionally substituted C₁₋₄ alkyl group. Suitably, the ester or nitrile group A undergoes base hydrolysis in this reaction.

Where A is an ester COOR², Formula I may be formed by hydrolysis and, where appropriate, deprotection at R¹ of a compound of Formula VIII:

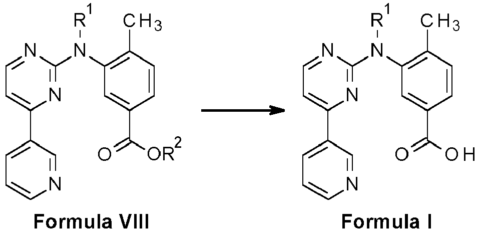

The alkyl group R² may be linear or branched. In some embodiments the alkyl is unsubstituted. In some embodiments the alkyl is substituted with, for example, halogen or optionally substituted aromatic groups, for example optionally substituted benzyl. Optionally substituted aromatic groups may be substituted with, for example, C₁₋₄alkyl (for example methyl), OH, OC₁₋₄alkyl (for example, OMe) and halogen.

For example, in some embodiments R² is methyl or ethyl.

The inventors provide two synthetic routes to Formula Villa.

In the first, a compound of Formula Villa is formed by condensation of a compound with Formula Vla with a compound of Formula VII: 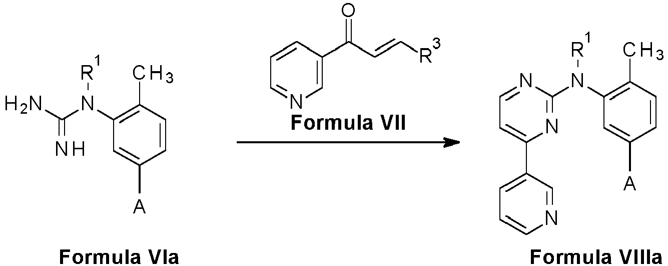 wherein Formula Vla is the free base or a chloride, bromide, iodide, mesylate, sulfate, phosphate, nitrate, acetate, oxalate, citrate, or tartrate salt; A is COOR² or CN; R¹ is H or a nitrogen protecting group, suitably H; R² is optionally substituted C₁₋₄alkyl group; and R³ is a suitable leaving group, suitably dialkyl-substituted nitrogen, for example NMe₂.

In some embodiments, A is CN.

Where A is COOR², a compound of Formula VIII is formed by condensation of a compound with Formula VI with a compound of Formula VII:

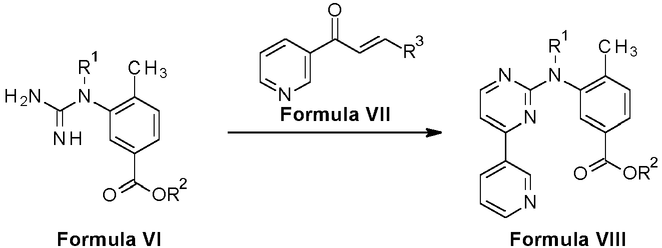

The reaction conditions may be based on those described in Zhongguo Yiyao Gongye Zazhi, 2009, 40, 401-403 which uses sodium hydroxide in ethanol heated at reflux and the nitrate salt of ethyl 3-guanidino-4-methylbenzoate, as described herein.

The reaction is suitably carried out under basic conditions, suitably with base with pKa > 13, more suitably, a hydroxide base. In some cases the base is sodium hydroxide. Suitable solvents are polar protic solvents, more suitably linear or branched C₁₋₄ alcohols, for example *n*-butanol or *tert*-butanol.

Suitably, the reaction is heated, for example at reflux.

The compound of Formula Vla may be synthesised from a compound of Formula Va:

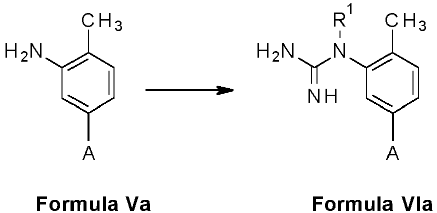

Thus, where A is COOR², the compound of Formula VI may be synthesised from a compound of Formula V:

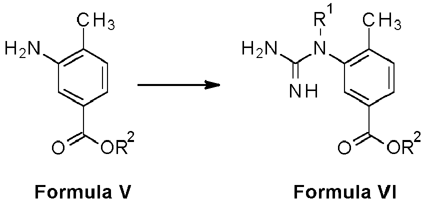

This reaction can be carried out using known guanylation techniques. One such technique uses cyanamide and acid, suitably an acid with pKa < 1, for example hydrochloric acid, in a polar protic solvent such as linear or branched C₁₋₄alcohols, for example methanol. Ammonium chloride or ammonium acetate may be added to yield the corresponding salt of the guanidine without use of explosive ammonium nitrate and to prevent hydrolysis of the ester under acidic conditions.

Another possible technique is using known guanyl-transfer reagents, for example *bis*-Boc-guanylpyrazole, 1-[*N*,*N*'-(di-Cbz)amidino]pyrazole, *N*,*N*=di-Boc-(*S*)-methylisothiourea, or 1,3-di-Boc-thiourea and subsequent deprotection of Boc groups under acidic conditions.

In an alternative method for the synthesis of Formula la, a compound of Formula XI is coupled to a compound of Formula Xlla: 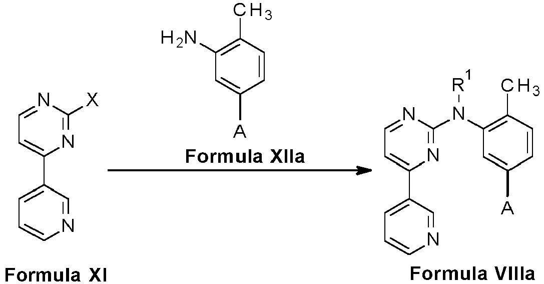 wherein X is a halogen, A is COOR² or CN, and R² is a linear or branched C₁₋₄ alkyl group.

Thus, where A is COOR², a compound of Formula XI is coupled to a compound of Formula XII:

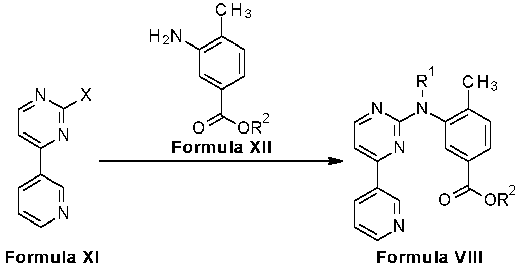

Suitable conditions for this reaction are acidic, suitably with catalytic amounts of an acid, suitably in the range of 0.1 to 0.4 eq., for example with an acid with pKₐ < 5, for example hydrochloric acid, acetic acid or methane sulfonic acid. Alternatively, the reaction may be palladium-catalysed (for example, using Pd₂dba₃ and a ligand such as BINAP) in basic conditions (for example, using Cs₂CO₃).

The solvent may be a polar aprotic solvent such as dioxane.

R¹ is hydrogen or, where the reaction further comprises a step of protecting the nitrogen, R¹ is a nitrogen protecting group. For example, after coupling the product may be treated with Boc anhydride (di-*tert*-butyl dicarbonate) (R¹ H → Boc).

Where A is COOR², hydrolysing the ester group A affords a compound of Formula la:

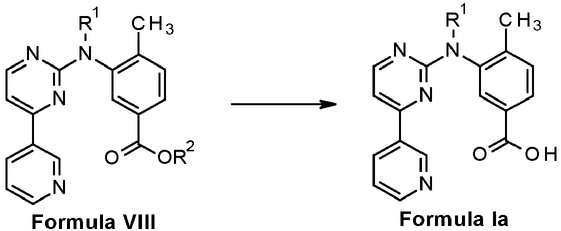

Analogously, where A is CN, hydrolysing the nitrile group A affords a compound of Formula la:

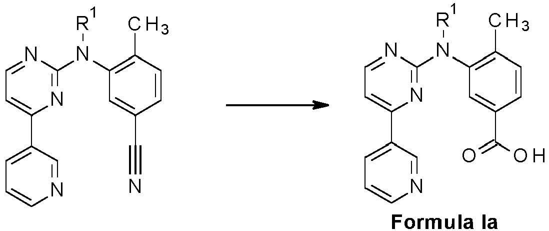

The compound of Formula XI may be synthesized via a cross coupling reaction with a compound of Formula IX: 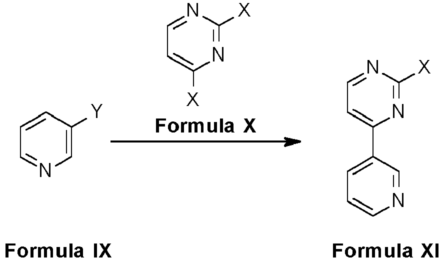 wherein each X is independently a halogen, and may also be a pseudohalide; and Y is a halogen, pseudohalide, or other group suitable for cross coupling, such as a trialkyltin group, a boronic acid group, or a boronic acid pinacol ester group. For example, the compound of Formula IX may be 3-bromopyridine. For example, the compound of Formula X may be 2,4-dichloropyrimidine. In this case, the compound of Formula XI is 2-chloro-4-(pyridin-3-yl)pyrimidine.

Suitable cross coupling reactions are known to a person skilled in the art and include Suzuki reaction (with palladium or nickel), Negishi reaction (with zinc), Stille reaction (with organotin), or Kumada coupling (via Grignard with palladium, nickel or iron). In the case where Kumada coupling conditions are employed, a Grignard reagent is made from Formula IX using magnesium that is activated by known techniques, for example heating, iodine, dibromoethane, or DIBAL-H. The catalyst used may suitably be an iron catalyst, for example Fe(acac)₃, FeCl₃, FeBr₃, or FeF₃.3H₂O with *N*,*N*'-(2,6-diisopropylphenyl)-dihydroimidazolium chloride

In some cases, the cross coupling is a Kumada coupling, the method comprising first forming a Grignard agent through treatment of the compound of Formula IX, for example, 3-bromopyridine followed by iron-catalyzed coupling with, for example Fe(acac)s.

In some cases, the cross coupling is a Suzuki reaction, the method comprising a palladium-catalysed coupling of a compound of Formula X with a boronic acid compound of Formula IXa (where Y is -B(OH)₂ in Formula IX):

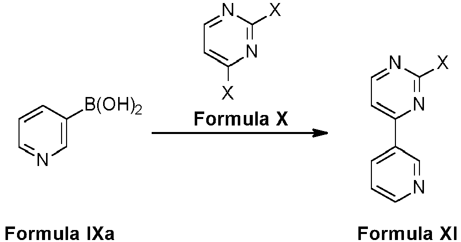

Alternatively, the Suzuki reaction can be carried out using a compound of Formula IX, in which Y is a boronic acid pinacol ester group:

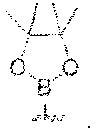

The palladium species in the Suzuki reaction may be, for instance, PdCl₂dppf·CH₂Cl₂. For example, the compound of Formula X may be 2,4-dichloropyrimidine. In this case, the compound of Formula XI is 2-chloro-4-(pyridin-3-yl)pyrimidine.

### Abbreviations

- Ar: argon gas
- BINAP: (2,2'-bis(diphenylphosphino)-1,1'-binaphthyl)
- Boc: *tert*-butyloxycarbonyl
- Bpoc: 2-(4-biphenyl)isopropoxycarbonyl
- CBz: carboxybenzyl
- dba: dibenzylideneacetone
- DBU: 1,8-diazabidcyclo[5.4.0]undec-7-ene
- DCM: dichloromethane
- Ddz: α,α-dimethyl-3,5-dimethoxybenzyloxycarbonyl
- DIPEA: *N*,*N*-diisopropylethylamine
- DMAP: *N*,*N*-dimethylpyridin-4-amine
- DMF: dimethylformamide
- DPPA: diphenylphosphoryl azide
- dppf: 1,1'-bis(diphenylphosphino)ferrocene
- EDC: 1-ethyl-3-(3-dimethylaminopropyl)carbodiimide
- Fmoc: 9-fluorenylmethoxycarbonyl
- HBTU: *N*,*N*,*N*',*N*'-tetramethyl-O-(1*H*-benzotriazol-1-yl)uronium hexafluorophosphate
- HOBt: hydroxybenzotriazole
- Pd/C: palladium on carbon
- PMB: *p*-methoxybenzyl
- T3P: propanephosphonic acid anhydride
- TEA: triethylamine
- Tf: triflate; trifluoromethanesulfonate (CF₃SO₃-)
- TFA: trifluoroacetic acid
- THF: tetrahydrofuran

The features disclosed in the foregoing description, or in the following claims, or in the accompanying drawings, expressed in their specific forms or in terms of a means for performing the disclosed function, or a method or process for obtaining the disclosed results, as appropriate, may, separately, or in any combination of such features, be utilised for realising the invention in diverse forms thereof.

For the avoidance of any doubt, any theoretical explanations provided herein are provided for the purposes of improving the understanding of a reader. The inventors do not wish to be bound by any of these theoretical explanations.

Any section headings used herein are for organizational purposes only and are not to be construed as limiting the subject matter described.

Throughout this specification, including the claims which follow, unless the context requires otherwise, the word "comprise" and "include", and variations such as "comprises", "comprising", and "including" will be understood to imply the inclusion of a stated integer or step or group of integers or steps but not the exclusion of any other integer or step or group of integers or steps.

It must be noted that, as used in the specification and the appended claims, the singular forms "a," "an," and "the" include plural referents unless the context clearly dictates otherwise.

### Examples

The following examples describe the synthesis of compounds of Formula la and Formula IIIa and their use in the synthesis of nilotinib and imatinib.

### Example 1

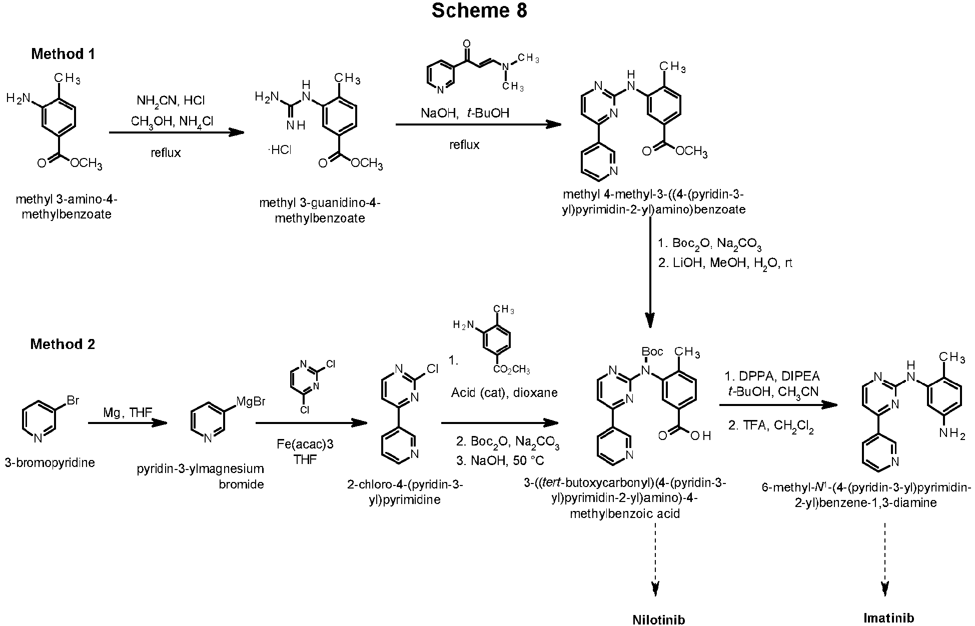

In Method 1 the inventors envisage a synthetic sequence via the conversion of commercially available methyl 3-amino-4-methylbenzoate to methyl 3-guanidino-4-methylbenzoate followed by condensation with 3-(dimethylamino)-1-(pyridin-3-yl)prop-2-en-1-one using *t*-butanol as solvent to afford methyl 4-methyl-3-((4-(pyridin-3-yl)pyrimidin-2-yl)amino)benzoate using modified conditions of the reported in Zhongguo Yiyao Gongye Zazhi, 2009, 40, 401-403.

In the modified conditions methyl and ethyl 3-guanidino-4-methylbenzoate will be prepared as the corresponding chloride salt to have better solubility and also to avoid the usage of explosive ammonium nitrate on a larger scale. Both the methyl and ethyl ester of 3-amino-4-ethylbenzoate will be investigated for comparison of reaction conditions with the corresponding alcohol (methanol for the methyl ester and ethanol for the ethyl ester) as the solvent to prevent transesterification. *Tert*-butanol will also be trialled as the solvent. For the condensation of 3-(dimethylamino)-1-(pyridin-3-yl)prop-2-en-1-one with methyl or ethyl 3-guanidino-4-methylbenzoate, bases such as potassium *tert*-butoxide, sodium methoxide and sodium ethoxide (commercially available or generated in situ) will be used.

Methyl or ethyl 4-methyl-3-((4-(pyridin-3-yl)pyrimidin-2-yl)amino)benzoate will then be *Boc*-protected using conventional conditions to provide 3-((*tert*-butoxycarbonyl)(4-(pyridin-3-yl)pyrimidin-2-yl)amino)-4-methylbenzoic acid. It will be appreciated that, as described herein, other acid labile protecting groups may be used in place of Boc. Suitable such groups may include α,α-dimethyl-3,5-dimethoxybenzyloxycarbonyl (Ddz), 2-(4-Biphenyl)isopropoxycarbonyl (Bpoc), *p*-methoxybenzyl (PMB), and 2,4-dimethoxybenzyl.

3-((*tert*-Butoxycarbonyl)(4-(pyridin-3-yl)pyrimidin-2-yl)amino)-4-methylbenzoic acid, or otherwise *N-*protected 3-((4-(pyridin-3-yl)pyrimidin-2-yl)amino)-4-methylbenzoic acid will then be converted into *tert-*butyl (5-((tert-butoxycarbonyl)amino)-2-methylphenyl)(4-(pyridin-3-yl)pyrimidin-2-yl)carbamate via a Curtius rearrangement using DPPA as the reagent and DIPEA as the base in acetonitrile and *t*-butanol as solvent (Scheme 9).

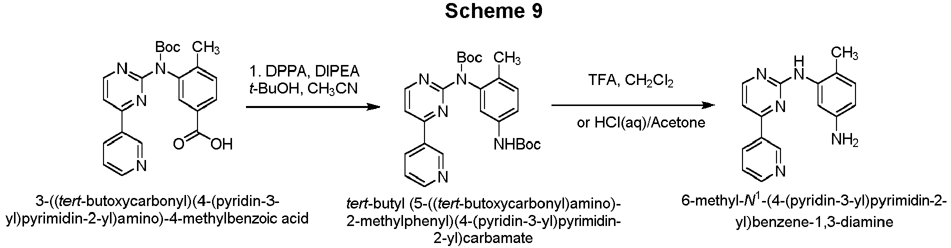

It will be appreciated that the nascent protecting group on the amino group of phenyl ring is a result of the choice of alcohol. By using different alcohols, other protecting groups will be obtained.

Various methods for deprotection of *tert*-butyl (5-((tert-butoxycarbonyl)amino)-2-methylphenyl)(4-(pyridin-3-yl)pyrimidin-2-yl)carbamate will be apparent to a person of skill in the art, and may include using the conventional trifluoroacetic acid in dichloromethane with control of the amount of trifluoroacetic acid. The inventors also envisage use of the method reported in Organic Process Research & Development 2004, 8, 945-947 using an aqueous solution of hydrochloric acid and acetone as solvent. In this case the compound of Formula III is expected to be isolated as the corresponding hydrochloride salt.

### Example 2

The synthesis of Nilotinib (Formula II) and Imatinib (Formula IV) was achieved through the synthesis of key advanced intermediate 4-methyl-3-((4-(pyridin-3-yl)pyrimidin-2-yl)amino)benzoic acid (Formula I) and 6-methyl-N1-(4-(pyridin-3-yl)pyrimidin-2-yl)benzene-1,3-diamine (Formula III) which were synthesized by 2 approaches as described in Scheme 10 and 11.

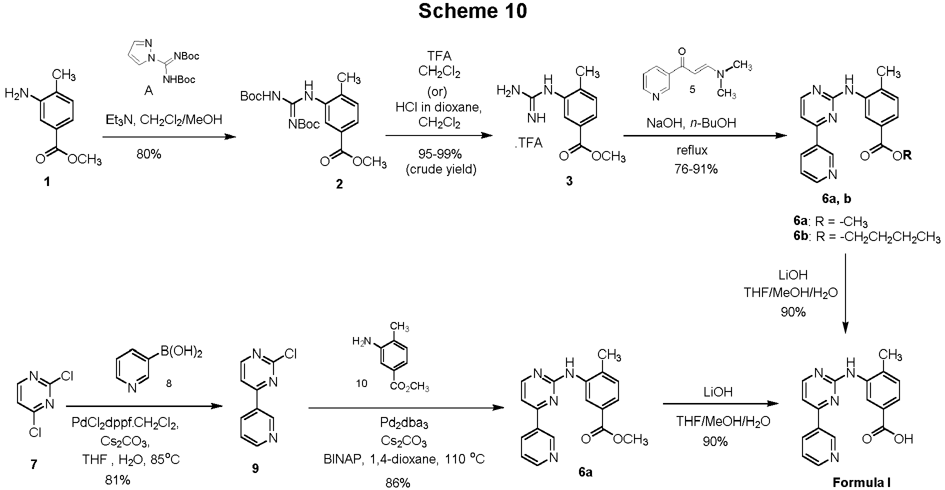

In Approach 1 as described in Scheme 10, Formula I was synthesized from two different methods from different starting materials. Thus methyl 3-amino-4-methylbenzoate (**1**) was subjected to guanylation using bis-Boc-guanyl pyrazole to afford methyl 3-(2,3-bis(*tert*-butoxycarbonyl)guanidino)-4-methylbenzoate (**3**). The Boc protecting groups were deprotected using either trifluoroacetic acid (TFA) or hydrochloric acid in 1 ,4-dioxane to afford the corresponding salt of methyl 3-guanidino-4-methylbenzoate (**4**) which was treated with 3-(dimethylamino)-1-(pyridin-3-yl)prop-2-en-1-one (**5**) to afford the corresponding esters methyl 4-methyl-3-((4-(pyridin-3-yl)pyrimidin-2-yl)amino)benzoate (**6a**) and/or butyl 4-methyl-3-((4-(pyridin-3-yl)pyrimidin-2-yl)amino)benzoate (**6b**). The trans-esterified product **6b** was the major product when the reaction time was prolonged or when the reaction temperature was 120 °C or slightly above. Both esters were smoothly hydrolyzed using lithium hydroxide to afford Formula I with good purity.

In another method, 2,4-dichloropyrimidine (**7**) was subjected to Suzuki coupling with 3-pyridyl boronic acid (**8**) using a modified literature protocol (Xin, Minhang et al, Bioorganic & Medicinal Chemistry Letters, 27(15), 3259-3263; 2017) to afford 2-chloro-4-(pyridin-3-yl)pyrimidine (**9**) as the exclusive product (conversion -90% and isolated yield 80%), while the formation of the other regioisomer or the bis adduct were not observed by TLC or HPLC analysis. 2-Chloro-4-(pyridin-3-yl)pyrimidine was then subjected to Buchwald coupling using Pd₂dba₃ as catalyst and BINAP as the ligand to afford methyl 4-methyl-3-((4-(pyridin-3-yl)pyrimidin-2-yl)amino)benzoate (**6a**) in good yield. **6a** was hydrolyzed using lithium hydroxide to afford Formula I.

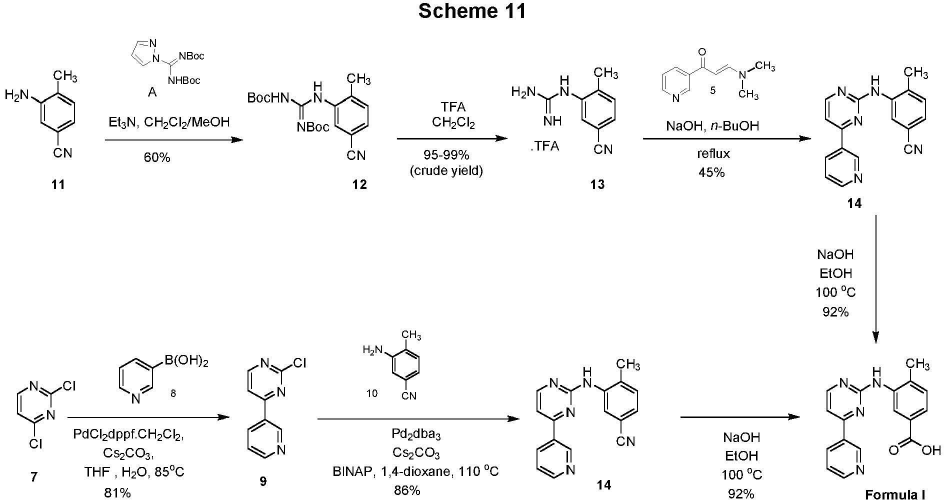

Approach 2 (Scheme 11) was a modified version of approach 1 with the nitrile-containing starting material replacing the ester functionality in both approaches. Thus in method 1, 3-amino-4-methylbenzonitrile (**11**) was subjected to guanylation using bis-Boc-guanyl pyrazole to afford methyl 3-(2,3-bis(*tert-*butoxycarbonyl)guanidino)-4-methylbenzonitrile (**12**) which was subjected to Boc deprotection using TFA to afford 1-(5-cyano-2-methylphenyl)guanidine (**13**). Treatment of **13** with the corresponding enaminone (**5**) afforded 4-methyl-3-((4-(pyridin-3-yl)pyrimidin-2-yl)amino)benzonitrile (**14**). Base hydrolysis of **14** using sodium hydroxide in ethanol under reflux conditions provided Formula I in excellent yields. Formula I was also obtained in high yields *via* Buchwald coupling of 2-chloro-4-(pyridin-3-yl)pyrimidine (**9**) with 3-amino-4-methylbenzonitrile (**11**) to afford **14** followed by basic hydrolysis (method 2). The other key intermediate *viz.* 6-methyl-N1-(4-(pyridin-3-yl)pyrimidin-2-yl)benzene-1,3-diamine (Formula III) was obtained from Formula I using a Curtius rearrangement in two methods (Scheme 12).

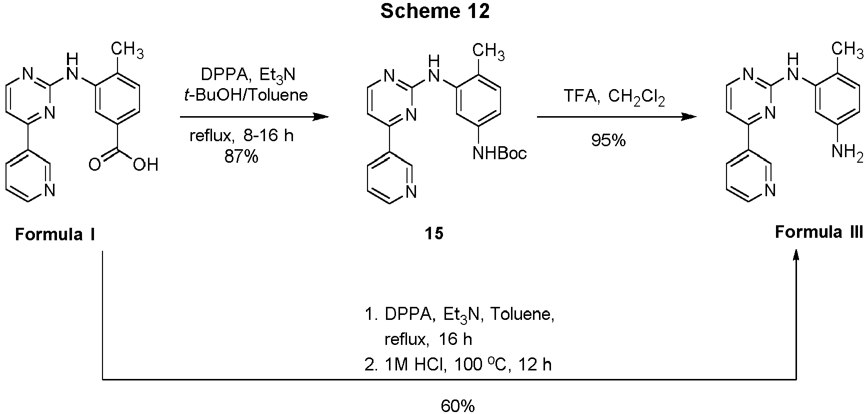

In one of the methods, Formula I was subjected to a one-pot Curtius rearrangement using DPPA, triethylamine and *t*-butanol/toluene (1:1) to afford *tert*-butyl (4-methyl-3-((4-(pyridin-3-yl)pyrimidin-2-yl)amino)phenyl)carbamate (15) which was easily deprotected using trifluoroacetic acid to give Formula III. In the other method Formula III was directly obtained after acid hydrolysis of the corresponding isocyanate intermediate (not isolated) that formed under Curtius rearrangement conditions using DPPA, triethylamine and toluene. However the amine was contaminated with phosphorous salt impurities (from DPPA) and the isolated yield was 60%. Hence the one-pot Curtius conditions to afford the corresponding carbamate was better in terms of yield and product purity.

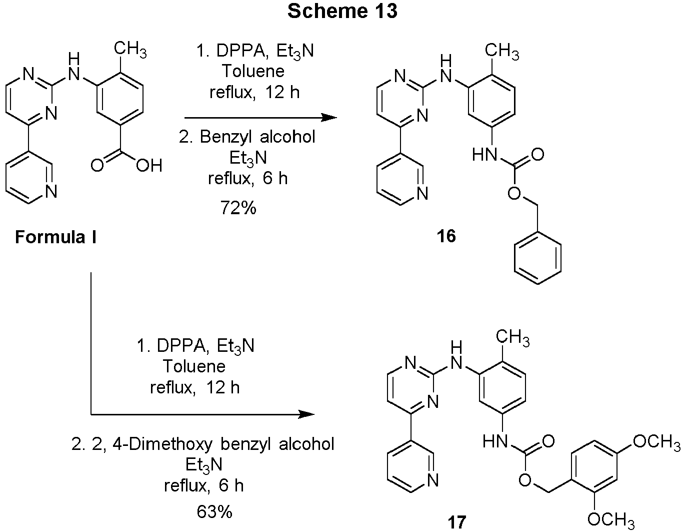

The conversion of Formula I to other carbamates *via* Curtius rearrangement was also explored, as shown in Scheme 13 above. Thus benzyl (4-methyl-3-((4-(pyridin-3-yl)pyrimidin-2-yl)amino) phenyl)carbamate (**16**) and 2,4-dimethoxybenzyl (4-methyl-3-((4-(pyridin-3-yl)pyrimidin-2-yl) amino) phenyl)carbamate (**17**) were obtained in good yields under the Curtius rearrangement conditions employed.

Nilotonib (Formula II) and Imatinib (Formula IV) were then obtained from Formula I (as acid chloride), **6a** (methyl ester) and **Formula III** respectively, as shown in Scheme 14.

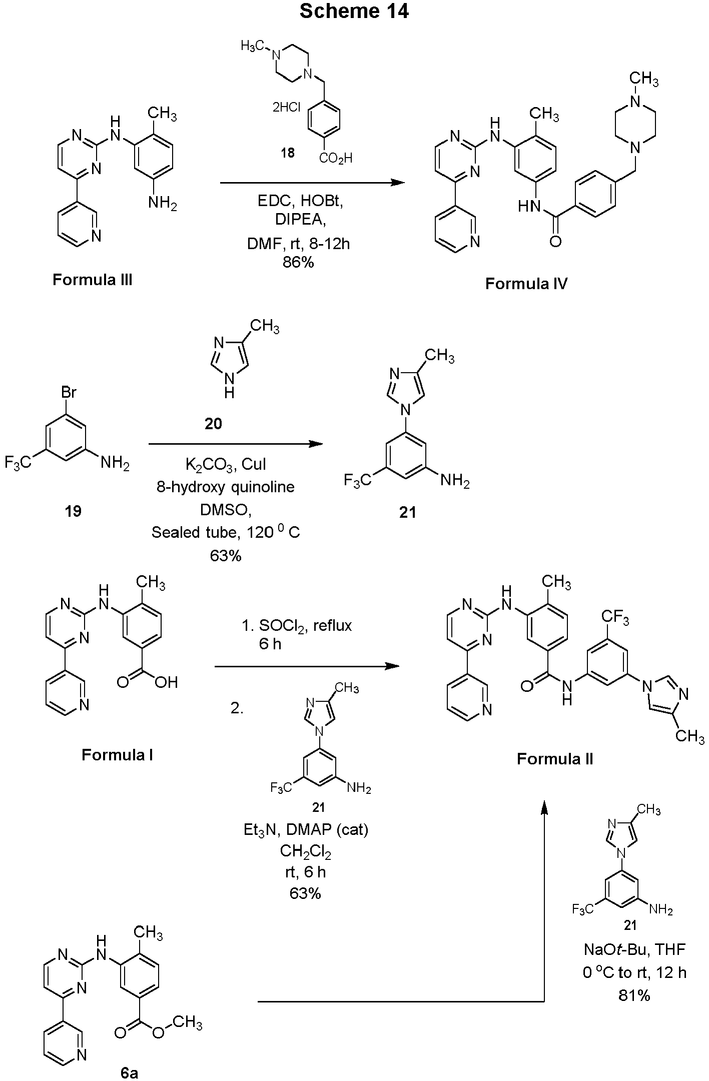

Thus the commercially available dihydrochloride salt of 4-((4-methylpiperazin-1-yl)methyl)benzoic acid (18) was treated with EDC, HOBt and DIPEA in DMF, followed by addition of Formula III at room temperature, which afforded *N*-(4-methyl-3-((4-(pyridin-3-yl)pyrimidin-2-yl)amino)phenyl)-4-((4-methylpiperazin-1-yl)methyl)benzamide (Imatinib; Formula IV) as an off-white solid in high yield.

On the other hand, similar treatment of Formula I with EDC, HOBt and DIPEA in DMF, followed by addition of 3-(4-methyl-1*H*-imidazol-1-yl)-5-(trifluoromethyl)aniline (**21**) (Huang, Wei-Sheng and Shakespeare, William C. Synthesis, (14), 2121-2124; 2007), only afforded the corresponding activated ester, which did not react further. Conversion of Formula I to the corresponding acid chloride using thionyl chloride under reflux conditions, followed by treatment with 3-(4-methyl-1*H*-imidazol-1-yl)-5-(trifluoromethyl)aniline (**21**), using triethylamine as base and a catalytic amout of DMAP, afforded Formula II in yields ranging from 60-70%. Meanwhile, the treatment of methyl 4-methyl-3-((4-(pyridin-3-yl)pyrimidin-2-yl)amino)benzoate (**6a**) with 3-(4-methyl-1*H*-imidazol-1-yl)-5-(trifluoromethyl)aniline, using sodium tert-butoxide as base under an argon atmosphere, gave a very clean reaction and afforded Formula II in better yields. However, 5-7 equivalents of sodium *tert*-butoxide were required to drive the reaction to completion. Overall, the sodium tert-butoxide mediated coupling of **6a** was better in terms of yield and isolation of product.

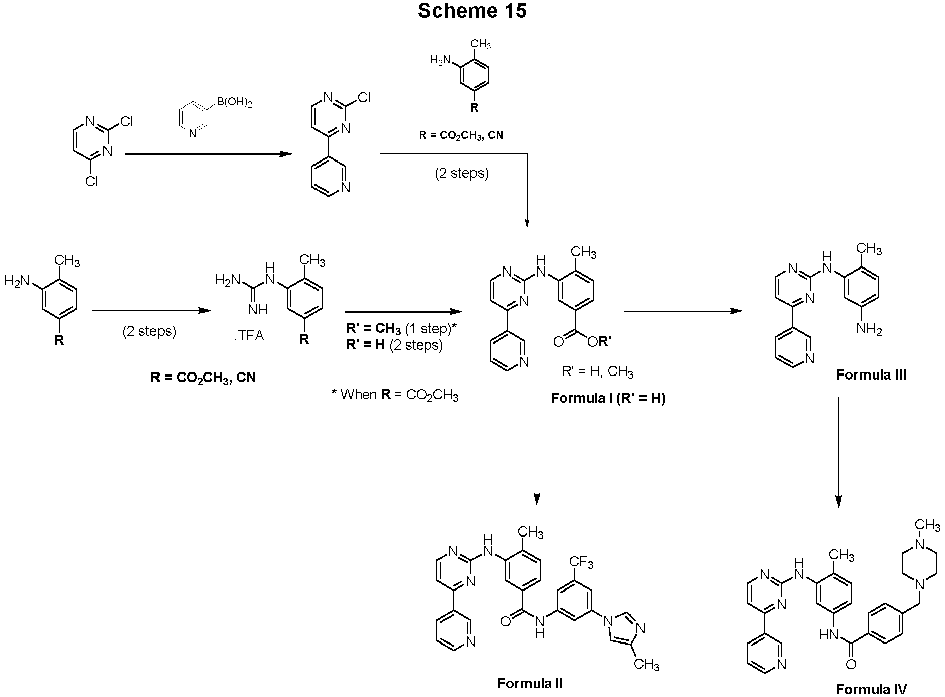

Thus a synthetic strategy (Scheme 15) with a single synthetic route (having 2 different approaches) for the synthesis of Formula I leading to Formula III via a Curtius rearrangement has been established, and hence the corresponding synthesis of Formula II and Formula IV has been made possible in this strategy. In particular, the reactions provide good yields and are scalable to enable bulk synthesis.

### General Experimental

Unless otherwise noted, reagents and solvents were used as received from commercial suppliers. Proton nuclear magnetic resonance spectra were obtained on a Bruker AVANCE 400 spectrometer at 400 MHz, tetramethylsilane was used as an internal standard for proton spectra. Thin-layer chromatography was performed using Merck TLC silica-gel 60F₂₅₄ plates. Visualization of TLC plates was performed using UV light (254 nm). The mass spectra were obtained on a Thermo LTQ Orbitrap spectrometer using electrospray ionization (ESI). HPLC analyses were performed on a Shimadzu Prominence LC-2030C 3D using HPLC Method 1 and Method 2 below. Column chromatographic purifications were carried out on a Biotage Selekt Flash purification system.

### Method 1

| | |
|---|---|
| Column: | Shim-pack GIST C18 (4.6 × 150 mm, 5.0 µm) |
| Column Temperature: | Ambient |
| Detection: | UV @ 254 nm |
| Sample Diluent: | Acetonitrile |
| Mobile Phase A: | Water |
| Mobile Phase B: | Acetonitrile with 0.05% TFA |

### Method 1 Gradient

| **Time (Minutes)** | **Flow (ml/min)** | **% Mobile Phase A** | **% Mobile Phase B** |
|---|---|---|---|
| 0:01 | 1.0 | 90 | 10 |
| 2.50 | 1.0 | 90 | 10 |
| 10:00 | 1.0 | 10 | 90 |
| 15:00 | 1.0 | 10 | 90 |
| 17:50 | 1.0 | 90 | 10 |
| 20:00 | Controller | Stop | |

### Method 2

| | |
|---|---|
| Column: | Shim-pack GIST C18 (4.6 × 150 mm, 5.0 µm) |
| Column Temperature: | Ambient |
| Detection: | UV @ 220 nm |
| Sample Diluent: | Acetonitrile |
| Mobile Phase A: | Water |
| Mobile Phase B: | Acetonitrile with 0.05% TFA |

### Method 2 Gradient

| **Time (Minutes)** | **Flow (ml/min)** | **% Mobile Phase A** | **% Mobile Phase B** |
|---|---|---|---|
| 0:01 | 1.0 | 95 | 5 |
| 7:50 | 1.0 | 90 | 10 |
| 12:50 | 1.0 | 30 | 70 |
| 14:00 | 1.0 | 10 | 90 |
| 16.50 | 1.0 | 10 | 90 |
| 17.00 | 1.0 | 90 | 10 |
| 20:00 | Controller | Stop | |

### Experimental - Methods of Synthesis & Data

### Synthesis of methyl-3-(2,3-bis(tert-butoxycarbonyl)guanidino)-4-methylbenzoate (2)

To a solution of methyl 3-amino-4-methylbenzoate (**1**) (2.0 g, 12.1 mmol) in MeOH/DCM (1:1, 50 ml), triethylamine (8.6 ml, 60.5 mmol) and *N*,*N*'-bis-Boc-1-guanylpyrazole (7.5 g, 24.2 mmol) were added. The reaction mixture was stirred at 40 °C for 24 h and then concentrated. The residue was treated with a large excess of water, filtered and the solid obtained was purified on a Biotage Selekt flash purification system (5-10% EtOAc/Hexanes) to afford methyl-3-(2,3-bis(*tert*-butoxycarbonyl)guanidino)-4-methylbenzoate (**2**) as a white solid (3.9 g, 80% yield). ¹H NMR (400 MHz, CDCl₃) δ ppm 11.71 (bs, 1H), 10.68 (s, 1H), 10.22 (bs, 1H), 7.78 (dd, J = 8.0, 2.0 Hz, 1H), 7.28 (d, J = 8.0 Hz, 1H), 3.92 (s, 3H), 2.38 (s, 3H), 1.54 (s, 9H), 1.51 (s, 9H). ESI-MS *m*/*z* 408 [M + H]⁺. HPLC; Rt (min): 12.33 (Method 1).

### Synthesis of methyl 3-guanidino-4-methylbenzoate trifluoroacetate salt (3)

To a solution of methyl-3-(2,3-bis(*tert*-butoxycarbonyl)guanidino)-4-methylbenzoate (**2**, 2.0 g, 4.9 mmol) in dichloromethane (20 ml), TFA (10 ml) was added dropwise and the reaction mixture was stirred at room temperature for 12 h and the progress of the reaction was monitored by HPLC analysis. The reaction mixture was concentrated *in vacuo* and the residue was dried to afford a colorless oil (1.49 g, 99% crude yield), which was taken forward to next step without further purification. ¹H NMR (400 MHz, CDCl₃) δ ppm 9.18 (bs, 2H), 8.50 (bs, 3H), 7.99 (dd, J = 8.0, 2.4 Hz, 1H), 7.89 (s, 1H), 7.46 (d, J = 8.0 Hz, 1H), 3.92 (s, 3H), 2.38 (s, 3H). ESI-MS *m*/*z* 208 [M + H]⁺. HPLC; Rt (min): 2.62 (Method 1).

### Synthesis of methyl 3-guanidino-4-methylbenzoate hydrochloride salt (3a)

To a solution of methyl-3-(2,3-bis(tert-butoxycarbonyl)guanidino)-4-methylbenzoate (1.0 g, 2.46 mmol) in dichloromethane (10 ml), HCl in dioxane (4M, 1.25 ml, 5.0 mmol) was added and the reaction mixture was stirred at room temperature for 12 h, and the progress of the reaction was monitored by HPLC analysis. The reaction mixture was concentrated *in vacuo* and the pale yellow oily residue (**3a**) was dried and used without further purification.

### Synthesis of butyl 4-methyl-3-((4-(pyridin-3-yl)pyrimidin-2-yl)amino)benzoate (6b)*

To a mixture of 3-guanidino-4-methylbenzoate trifluoroacetate salt (**3**) (1.4 g, 4.60 mmol) and 3-(dimethylamino)-1-(pyridin-3-yl)prop-2-en-1-one (970 mg, 5.5 mmol) in *n*-BuOH (10 ml), a solution of sodium hydroxide (370 mg, 9.2 mmol) in *n*-BuOH (5 ml) was added. The reaction mixture was heated to reflux at 120 °C for 24 h*and then concentrated *in vacuo* to remove the solvent. The residue was partioned between H₂O (20 ml) and ethyl acetate (20 ml), and the aqeous layer was extracted with ethyl acetate (2 × 15 ml). The combined organic layer was dried (Na₂SO₄), concentrated and the residue was purified on a Biotage Selekt flash purification system (silica gel, 3-5% MeOH in DCM), which afforded **6b** as a pale yellow oil (1.50 g, 90% yield). [Note: * mixture (1:2) of methyl (**6a**) and butyl esters (**6b**) were obtained after 12 h and when the temperatures was below 110 °C; heating to reflux for 24 h at 120 °C leads to the formation to n-butyl ester as the major product.] ¹H NMR (400 MHz, DMSO-*d₆*) δ ppm 9.29 (d, J = 1.6 Hz, 1H), 9.08 (s, 1H), 8.72 (dd, J = 4.8, 1.6 Hz, 1H), 8.57 (d, J = 4.8 Hz, 1H), 8.45 (dt, J = 8.0, 2.0 Hz, 1H), 8.36 (d, J = 1.2 Hz, 1H), 7.67 (dd, J = 8.0, 2.0 Hz, 1H), 7.56 (dd, J = 8.0, 4.8 Hz, 1H), 7.51 (d, J = 4.8 Hz, 1H), 7.41 (d, J = 8.0 Hz, 1H), 4.29 (t, J = 6.4 Hz, 2H), 1.7 (q, J = 2.8 Hz, 2H), 1.43 (q, J = 7.6 Hz, 2H), 0.89 (t, J = 7.6 Hz, 3H). ESI-MS *m*/*z* 363 [M + H]⁺. HPLC; Rₜ (min) : 10.22 (Method 1).

### Synthesis of 4-methyl-3-((4-(pyridin-3-yl)pyrimidin-2-yl)amino)benzoic acid (Formula I)

To a mixture of butyl 4-methyl-3-((4-(pyridin-3-yl)pyrimidin-2-yl)amino)benzoate (**6b**, 1.48 g, 4.1 mmol) in THF/MeOH/H₂O (15 ml), LiOH (860 mg, 20.5 mmol) was added. The reaction mixture was stirred at room temperature for 12 h. After completion of the reaction, the reaction mixture was concentrated *in vacuo* and the residue was suspended in H₂O (15 ml). The pH of the solution was adjusted to 7.0 using hydrochloric acid (1M, aq) and the precipitated pale yellow solid was filtered and dried to afford 4-methyl-3-((4-(pyridin-3-yl)pyrimidin-2-yl)amino)benzoic acid (1.13 g, 90%). ¹H NMR (400 MHz, DMSO-*d₆*) δ ppm 9.29 (d J = 2.4 Hz, 1H), 9.06 (s, 1H), 8.71 (dd, J = 4.8, 1.6 Hz, 1H), 8.56 (d, J = 4.8 Hz, 1H), 8.48 (dt, J = 8.0, 2.0 Hz, 1H), 8.32 (d, J = 1.6 Hz, 1H), 7.66 (dd, J = 7.6, 1.6 Hz, 1H), 7.55 (dd, J = 8.0, 4.8 Hz, 1H), 7.49 (d, J = 5.2 Hz, 1H), 7.38 (d, J = 8.0 Hz, 1H), 2.34 (s, 3H). ESI-MS *m*/*z* 307 [M + H]⁺. HPLC; Rt (min): 7.82 (Method 1).

### Synthesis of 2-chloro-4-(pyridin-3-yl)pyrimidine (9)

A mixture of 2,4-dichloropyrimidine (**7**, 2.0 g, 13. 4 mmol), pyridin-3-ylboronic acid (**8**, 1.65 g 13.4 mmol), Cs₂CO₃ (14.2 g, 40. 3 mmol) in THF (45 ml) and H₂O (30 ml) was purged under Ar for about 10 minutes. PdCl₂dppf.CH₂Cl₂ (650 mg, 0.81 mmol) was added to the reaction mixture and purged with Ar for another 5 minutes. The reaction mixture was then heated at 85 °C for 12 h. After completion of the reaction, the reaction mixture was cooled to room temperature and passed through a short Celite^{®} pad. The filtrate was partioned between EtOAc (60 ml) and H₂O (30 ml). The aq layer was extracted with EtOAc (2 × 30 ml) and the combined organic layer was dried (Na₂SO₄) and concentrated, and the residue was purified on a Biotage Selekt flash purification system (silica gel, 3-5% MeOH/DCM), which afforded 2-chloro-4-(pyridin-3-yl)pyrimidine (**9**) as an off-white solid (2.08 g, 81%). ¹H NMR (400 MHz, DMSO-*d₆*) δ ppm 9.36 (d, J = 2.4 Hz, 1H), 8.91 (d, J = 5.2 Hz, 1H), 8.79 (dd, J = 4.8, 1.6 Hz, 1H), 8.56 (dt, J = 8.0, 2.4 Hz, 1H), 8.27 (d, J = 5.2 Hz, 1H), 7.63 (dd J = 8.0, 4.8 Hz, 1H). ESI-MS *m*/*z* 192 [M + H]⁺. HPLC: Rt (min): 7.39 (Method 1).

### Synthesis of methyl 4-methyl-3-((4-(pyridin-3-yl)pyrimidin-2-yl)amino)benzoate (6a)

A mixture of 2-chloro-4-(pyridin-3-yl)pyrimidine (**9**, 1.0 g, 5.23 mmol), methyl 3-amino-4-methylbenzoate (**10**, 1.12 g, 6.8 mmol), Cs₂CO₃ (3.4 g, 10.5 mmol), BINAP (390 mg, 0.63 mmol) in 1,4-dioxane (40 ml) was purged under Ar for 10 minutes. Pd₂dba₃ (240 mg, 0.26 mmol) was added and the reaction mixture was purged for another 5 minutes. The reaction mixture was heated at 100 °C for 12-20 h until (**9**) was completely consumed (HPLC analysis). The reaction mixture was cooled to room temperature and passed through a short Celite^{®} pad and the filtrate was partioned between EtOAc (40 ml) and H₂O (20 ml). The aq layer was extracted with EtOAc (2 × 25 ml), and the combined organic layer was dried (Na₂SO₄), concentrated and the residue was purified on a Biotage Selekt flash purification system (silica gel, 3-5% MeOH/DCM), which afforded methyl 4-methyl-3-((4-(pyridin-3-yl)pyrimidin-2-yl)amino) benzoate **(6a)** as a pale brown solid (1.44 g, 86%). ¹H NMR (400 MHz, DMSO-*d₆*) δ ppm 9.31 (d, J = 1.6 Hz, 1H), 9.06 (s, 1H), 8.72 (dd, J = 4.8, 1.6 Hz, 1H), 8.58 (d, J = 4.8 Hz, 1H), 8.48 (dt, J = 8.0, 2.0 Hz, 1H), 8.42 (d, J = 1.2 Hz, 1H), 7.67 (dd, J = 8.0, 2.0 Hz, 1H), 7.58 (dd, J = 8.0, 2.0 Hz, 1H), 7.52 (d, J = 5.2 Hz, 1H), 7.41 (d, J = 8.0 Hz, 1H), 3.87 (s, 3H), 2.49 (s, 3H). ESI-MS *m*/*z* 319 [M-H]⁻. HPLC: Rt (min) : 8.83 (Method 1).

### Synthesis of methyl 3-(2,3-bis(tert-butoxycarbonyl)guanidino)-4-methylbenzonitrile (12)

To a solution of methyl 3-amino-4-methylbenzonitrile (**11**) (1.0 g, 7.56 mmol) in MeOH/DCM (1:1, 30 ml), triethylamine (8.6 ml, 60.5 mmol) and *N*,*N*'-bis-Boc-1-guanylpyrazole (4.7 g, 15.1 mmol) were added. The reaction mixture was stirred at 50 °C for 24 h and then concentrated. The residue was treated with large excess of water, filtered and the solid obtained was purified on a Biotage Selekt flash purification system (Silica gel, 5-10% Ethyl acetate/Hexanes) to afford methyl -3-(2,3-bis(tert-butoxycarbonyl)guanidino)-4-methylbenzonitrile (**12**) as a white solid (1.7 g, 60% yield). ¹H NMR (400 MHz, CDCl₃) δ ppm 11.69 (s, 1H), 10. 37 (s, 1H), 8.49 (s, 1H), 7.36 (dd, J = 8.0, 2.0 Hz, 1H), 7.28 (d, J = 8.0 Hz, 1H), 2.39 (s, 3H), 1. 54 (s, 9H), 1.52 (s, 9H). ESI-MS *m*/*z* 375 [M + H]⁺. HPLC: Rₜ(min): 12.62 (Method 1).

### Synthesis of 1-(5-cyano-2-methylphenyl)guanidine trifluoroacetate salt (13)

To a solution of methyl 3-(2,3-bis(tert-butoxycarbonyl)guanidino)-4-methylbenzonitrile (**12**, 1.0 g, 2.67 mmol) in dichloromethane (10 ml), TFA (5 ml) was added dropwise and the reaction mixture was stirred at room temperature for 12 h, and the progress of the reaction was monitored by HPLC analysis. The reaction mixture was concentrated *in vacuo* and the residue was dried to afford a colorless oil (**13**) (726 mg, 99% crude yield), which was taken forward to the next step without further purification. ¹H NMR (400 MHz, DMSO-*d₆*) δ ppm 9.75 (s, 0.5H), 9.63 (s, 0.5H), 7.78-7.70 (m, 2H), 7.56 (s, 1H), 7.54 (bs, 2H), 7.43 (bs, 1H), 7.41 (d, J = 8.0 Hz, 1H), 2.29 (d, J = 10.0 Hz, 3H). HPLC; Rt (min): 2.73 (Method 1).

### Synthesis of 4-methyl-3-((4-(pyridin-3-yl)pyrimidin-2-yl)amino)benzonitrile (14)

To a mixture of 1-(5-cyano-2-methylphenyl)guanidine trifluoroacetate salt (720 mg, 2.65 mmol) and 3-(dimethylamino)-1-(pyridin-3-yl)prop-2-en-1-one (560 mg 3.18 mmol) in n-BuOH (10 ml), a solution of sodium hydroxide (220 mg, 5.3 mmol) in n-BuOH (5 ml) was added. The reaction mixture was heated to reflux at 120 °C for 24 h and then concentrated *in vacuo* to remove the solvent. The residue was partioned between H₂O (20 ml) and ethyl acetate (20 ml), and the aqeous layer was extracted with ethyl acetate (2 × 15 ml). The combined organic layer was dried (Na₂SO₄) and concentrated, and the residue was purified on a Biotage Selekt flash purification system (silica gel, 3-5% MeOH in DCM), which afforded 4-methyl-3-((4-(pyridin-3-yl)pyrimidin-2-yl)amino)benzonitrile (**14**) as a pale brown solid (342 mg, 45%).

### Synthesis of 4-methyl-3-((4-(pyridin-3-yl)pyrimidin-2-yl)amino)benzonitrile (14) using Buchwald coupling

A mixture of 2-chloro-4-(pyridin-3-yl)pyrimidine (**9**, 1.0 g, 5.23 mmol), 3-amino-4-methylbenzonitrile (900 mg, 6.8 mmol), Cs₂CO₃ (3.4 g, 10.5 mmol), BINAP (390 mg, 0.63 mmol) in 1,4-dioxane (30 ml) was purged under Ar for 10 minutes. Pd₂dba₃ (240 mg, 0.26 mmol) was added and the reaction mixture was purged for another 5 minutes. The reaction mixture was heated at 100 °C for 12-20 h until (**9**) was completely consumed (HPLC analysis). The reaction mixture was cooled to room temperature and passed through a short Celite^{®} pad, and the filtrate was partioned between EtOAc (30 ml) and H₂O (20 ml). The aq layer was extracted with EtOAc (2 × 20 ml), and the combined organic layer was dried (Na₂SO₄), concentrated and the residue was purified on a Biotage Selekt flash purification system (silica gel, 3-5% MeOH DCM), which afforded 4-methyl-3-((4-(pyridin-3-yl)pyrimidin-2-yl)amino)benzonitrile (**14**) as a pale yellow-brown solid (1.29 g, 86%). ¹H NMR (400 MHz, DMSO-*d₆*) δ ppm 9.28 (d, J = 2.0 Hz, 1H), 9.17 (s, 1H), 8.73 (dd, J = 8.4, 1.6 Hz, 1H), 8.59 (d, J = 5.2 Hz, 1H), 8.44 (dt, J = 8.0, 1.6 Hz, 1H), 8.12 (d, J = 1.2 Hz, 1H), 7.58 (dd, J = 8.0, 4.8 Hz, 1H), 7.54 - 7.50 (m, 2H), 7.47 (d, J = 8.0 Hz, 1H), 2.37 (s, 3H). ESI-MS *m*/*z* 288 [M + H]⁺. HPLC; Rt (min): 8.63 (Method 1).

### Synthesis of 4-methyl-3-((4-(pyridin-3-yl)pyrimidin-2-yl)amino)benzoic acid (Formula I)

To a solution of 4-methyl-3-((4-(pyridin-3-yl)pyrimidin-2-yl)amino)benzonitrile (**14**, 800 mg, 2.79 mmol) in ethanol (15 ml), NaOH (1M (aq), 11.1 ml) was added. The reaction mixture was heated at 100 °C for 5 h. The reaction mixture was concentrated and diluted with H₂O (15 ml) and the pH was adjusted to 7.0 (neutral). The precipitated yellow solid was filtered and dried to afford 4-methyl-3-((4-(pyridin-3-yl)pyrimidin-2-yl)amino)benzoic acid (783 mg, 92%). ¹H NMR (400 MHz, DMSO-*d₆*) δ ppm 9.29 (d J = 2.4 Hz, 1H), 9.06 (s, 1H), 8.71 (dd, J = 4.8, 1.6 Hz, 1H), 8.56 (d, J = 4.8 Hz, 1H), 8.48 (dt, J = 8.0, 2.0 Hz, 1H), 8.32 (d, J = 1.6 Hz, 1H), 7.66 (dd, J = 7.6, 1.6 Hz, 1H), 7.55 (dd, J = 8.0, 4.8 Hz, 1H), 7.49 (d, J = 5.2 Hz, 1H), 7.38 (d, J = 8.0 Hz, 1H), 2.34 (s, 3H). ESI-MS *m*/*z* 307 [M + H]⁺. HPLC; Rₜ(min): 7.82 (Method 1).

### Synthesis of tert-butyl (4-methyl-3-((4-(pyridin-3-yl)pyrimidin-2-yl)amino)phenyl) carbamate (15)

To a mixture of 4-methyl-3-((4-(pyridin-3-yl)pyrimidin-2-yl)amino)benzoic acid (Formula I, 2.0 g, 6.53 mmol) in t-BuOH/toluene (1:1, 40 ml), triethylamine (1.36 ml, 9.8 mmol) followed by DPPA (2.12 ml, 9.8 mmol) were added. The reaction mixture was heated at 100 °C for 12 h. The reaction mixture was concentrated and the residue was partitioned between CH₂Cl₂ (40 ml) and H₂O (20 ml). The aqueous layer was extracted with CH₂Cl₂ (2 × 20 ml). The combined organic layers were dried (Na₂SO₄), concentrated and the residue was purified on a Biotage Selekt flash purification system (silica gel, 3-5% MeOH/DCM) to afford *tert*-butyl (4-methyl-3-((4-(pyridin-3-yl)pyrimidin-2-yl)amino)phenyl)carbamate (**15**) as a pale yellow foam (2.16 g, 87%). ¹H NMR (400 MHz, DMSO-*d₆*) δ ppm 9.27 (bs, 1H), 9.26 (s, 1H), 8.87 (s, 1H), 8.70 (dd, J = 4.8, 1.2 Hz, 1H), 8.51 (d, J = 5.2 Hz, 1H), 8.49 (dt, J = 8.0, 2.0 Hz, 1H), 7.80 (s, 1H), 7.53 (dd, J = 8.0, 4.8 Hz, 1H), 7.42 (d, J = 5.2 Hz, 1H), 7.14 (dd, J = 8.0, 1.6 Hz, 1H), 7.10 (d, J = 8.0 Hz, 1H), 2.17 (s, 3H), 1.47 (s, 3H). ESI-MS *m*/*z* 378 [M + H]⁺; 400 [M + Na]⁺. HPLC; Rt: (min): 9.43 (Method 1).

### Synthesis of 6-methyl-N1-(4-(pyridin-3-yl)pyrimidin-2-yl)benzene-1,3-diamine (Formula III)

To a solution of *tert*-butyl (4-methyl-3-((4-(pyridin-3-yl)pyrimidin-2-yl)amino)phenyl) carbamate (**15**) (1.0 g, 2.65 mmol) in CH₂Cl₂(10 ml), trifluoracetic acid (5 ml) was added and the reaction mixture was stirred for 8 h. The reaction mixture was concentrated *in vacuo* and the residue was suspended in H₂O (10 ml). NaOH (1M, aq) was added to this suspension and the pH was adjusted to >8.0 and then extracted with CH₂Cl₂ (2 × 25 ml). The CH₂Cl₂ layer was dried (Na₂SO₄) and concentrated to afford 6-methyl-N1-(4-(pyridin-3-yl)pyrimidin-2-yl)benzene-1,3-diamine (Formula III) as a yellow solid (698 mg, 95%). ¹H NMR (400 MHz, DMSO-*d₆*) δ ppm 9.26 (d, J = 2.0 Hz, 1H), 8.70 (dd, J = 4.8, 1.2 Hz, 1H), 8.67 (s, 1H), 8.48 (d, J = 5.2 Hz, 1H), 8.43 (dt, J = 8.0, 2.0 Hz, 1H), 7.56 (dd, J = = 8.0, 4.8 Hz, 1H), 7.37 (d, J = 4.8 Hz, 1H), 6.88 (d, J = 8.0 Hz, 1H), 6.8 (d, J = 2.0 Hz, 1H), 6.35 (dd, J = 8.0, 2.4 Hz, 1H), 4.85 (bs, 2H), 2.07 (s, 3H). ESI-MS *m*/*z* 278 [M + H]⁺. HPLC; Rₜ(min): 2.69 (Method 1).

### Synthesis of 6-methyl-N1-(4-(pyridin-3-yl)pyrimidin-2-yl)benzene-1,3-diamine (Formula III) - Curtius Rearrangement

To a mixture of 4-methyl-3-((4-(pyridin-3-yl)pyrimidin-2-yl)amino)benzoic acid (Formula I, 1.0 g, 3.26 mmol) in toluene (20 ml), triethylamine (0.7 ml, 4.9 mmol) followed by DPPA (1.1 ml, 4.9 mmol) were added. The reaction mixture was heated at 100 °C for 12 h. The reaction mixture was cooled to room temperature and 1M HCl (aq, 19 ml) was carefully added dropwise, and the reaction mixture was again heated at 100 °C for 6-8 h. The reaction mixture was cooled to room temperature and concentrated in vacuo to afford an aqueous suspension which was diluted with H₂O (15 ml). The pH was adjusted to >8.0 by a careful dropwise addition of 1M NaOH and then extracted with CH₂Cl₂ (2 × 30 ml). The CH₂Cl₂ layer was dried (Na₂SO₄), concentrated and purified on a Biotage Selekt flash purification system (silica gel, 3-5% MeOH/DCM with 0.1% EtsN) to afford 6-methyl-N1-(4-(pyridin-3-yl)pyrimidin-2-yl)benzene-1,3-diamine (Formula III) as a yellow solid (545 mg, 60% yield). Also isolated (200 mg) impure product contaminated with the phosponium salt. ¹H NMR (400 MHz, DMSO-*d₆*) δ ppm 9.26 (d, J = 2.0 Hz, 1H), 8.70 (dd, J = 4.8, 1.2 Hz, 1H), 8.67 (s, 1H), 8.48 (d, J = 5.2 Hz, 1H), 8.43 (dt, J = 8.0, 2.0 Hz, 1H), 7.56 (dd, J = = 8.0, 4.8 Hz, 1H), 7.37 (d, J = 4.8 Hz, 1H), 6.88 (d, J = 8.0 Hz, 1H), 6.8 (d, J = 2.0 Hz, 1H), 6.35 (dd, J = 8.0, 2.4 Hz, 1H), 4.85 (bs, 2H). 2.07 (s, 3H). ESI-MS *m*/*z m*/*z* 278 [M + H]⁺. HPLC; Rₜ(min): 2.69 (Method 1).

### Synthesis of benzyl (4-methyl-3-((4-(pyridin-3-yl)pyrimidin-2-yl)amino)phenyl)carbamate (16)

To a mixture of 4-methyl-3-((4-(pyridin-3-yl)pyrimidin-2-yl)amino)benzoic acid (Formula I, 310 mg, 1.0 mmol) in toluene (10 ml), triethylamine (0.2 ml, 1.5 mmol), DPPA (0.3 ml, 1.5 mmol) were added. The reaction mixture was heated at 100 °C for 12 h. The reaction mixture was cooled to room temperature and benzyl alcohol (0.5 ml, 5.0 mmol) and triethylamine (0.2 ml, 1.5 mmol) were added, and the reaction mixture was refluxed for another 12 h. The reaction mixture was concentrated and the residue was partitioned between CH₂Cl₂ (15 ml) and H₂O (10 ml). The aqueous layer was extracted with CH₂Cl₂ (2 × 10 ml). The combined organic layers were dried (Na₂SO₄), concentrated and the residue was purified on a Biotage Selekt flash purification system (silica gel, 3-5% MeOH/DCM) to afford benzyl (4-methyl-3-((4-(pyridin-3-yl)pyrimidin-2-yl)amino)phenyl)carbamate (**16**) as an off-white foam (296 mg, 72%). ¹H NMR (400 MHz, DMSO-*d₆*) δ ppm 9.71 (s, 1H), 9.27 (d, J = 2.0 Hz, 1H), 8.89 (s, 1H), 8.70 (dd, J = 8.8, 1.6 Hz, 1H), 8.51 (d, J = 4.8 Hz, 1H), 8.47 (dt, , J = 8.0, 2.0 Hz, 1H), 7.77 (s, 1H), 7.52 (dd, J = 8.0, 4.8 Hz, 1H), 7.44 - 7.36 (m, 7H), 7.20 - 7.12 (m, 2H), 5.15 (s, 2H), 2.18 (s, 3H). ESI-MS *m*/*z* 412 [M + H]⁺; 434 [M + Na]⁺. HPLC; Rₜ(min): 2.23 (Method 1).

### Synthesis of 2,4-dimethoxybenzyl (4-methyl-3-((4-(pyridin-3-yl)pyrimidin-2-yl)amino)phenyl) carbamate (17)

To a mixture of 4-methyl-3-((4-(pyridin-3-yl)pyrimidin-2-yl)amino)benzoic acid (Formula I, 310 mg, 1.0 mmol) in toluene (10 ml), triethylamine (0.2 ml, 1.5 mmol) and DPPA (0.3 ml, 1.5 mmol) were added. The reaction mixture was heated at 100 °C for 12 h. The reaction mixture was cooled to room temperature and 2,4-dimethoxybenzyl alcohol (420 mg, 2.5 mmol) and triethylamine (0.2 ml, 1.5 mmol) were added, and the reaction mixture was refluxed for another 12 h. The reaction mixture was concentrated and the residue was partitioned between CH₂Cl₂ (15 ml) and H₂O (10 ml). The aqueous layer was extracted with CH₂Cl₂ (2 × 10 ml). The combined organic layers were dried (Na₂SO₄), concentrated and the residue was purified on a Biotage Selekt flash purification system (silica gel, 3-5% MeOH/DCM) to afford 2,4-dimethoxybenzyl (4-methyl-3-((4-(pyridin-3-yl)pyrimidin-2-yl)amino)phenyl) carbamate (17) as a pale yellow solid (296 mg, 63%). ¹H NMR (400 MHz, DMSO-*d₆*) δ ppm 9.62 (s,1H), 9.26 (d, J = 1.6 Hz, 1H), 8.88 (s, 1H), 8.70 (dd, J = 4.4, 1.6 Hz, 1H), 8.50 (d, J = 5.2 Hz, 1H), 8.49 (dt, , J = 8.0, 2.0 Hz, 1H), 7.75-7.71 (m, 1H), 7.53 - 7.50 (m, 1H), 7.53 (dd, J = 7.6, 4.4 Hz, 1H), 7.42 (d, J = 4.8 Hz, 1H), 7.31 (d, J = 8.4 Hz, 1H), 7.19 (dd, J = 8.0, 1.6 Hz, 1H), 7.13 (d, J = 8.4 Hz, 1H), 6.59 (d, J = 2.4 Hz, 1H), 6.53 (d, J = 8.4, 2.0 Hz, 1H), 5.04 (s, 2H), 3.80 (s, 3H), 3.77 (s, 3H), 2.17 (s, 3H). ESI-MS *m*/*z* 472 [M + H]⁺. HPLC; Rt (min): 11.02 (Method 1).

### Synthesis of N-(4-methyl-3-((4-(pyridin-3-yl)pyrimidin-2-yl)amino)phenyl)-4-((4-methyl-piperazin-1-yl)methyl)benzamide (Imatinib, Formula IV)

### (a) Using DMF as solvent

To a solution of 4-((4-methylpiperazin-1-yl)methyl)benzoic acid dihydrochloride (**18**, 1.45 g, 4.70 mmol) in DMF (20 ml), DIPEA (4.0 ml, 23.5 mmol), EDC (1.08 g, 5.63 mmol) and HOBt (0.83 g, 6.1 mmol) were added and stirred for 10 minutes. 6-Methyl-N1-(4-(pyridin-3-yl)pyrimidin-2-yl)benzene-1,3-diamine (Formula III, 1.0 g, 3.6 mmol) was added and the reaction mixture was stirred at room temperature for 12 h. After completion of the reaction, the reaction mixture was poured into water (50 ml) and extracted with the EtOAc (2 × 30 ml). The EtOAc layer was dried (Na₂SO₄) and concentrated to afford the crude product which was triturated n-heptane and filtered. The filtered solid was washed with n-heptane containing a few drops of acetonitrile to afford *N*-(4-methyl-3-((4-(pyridin-3-yl)pyrimidin-2-yl)amino)phenyl)-4-((4-methyl-piperazin-1-yl)methyl)benzamide (Formula IV) as an off-white solid (1.53 g, 86%).

### (b) Using acetonitrile as solvent

To a solution of 4-((4-methylpiperazin-1-yl)methyl)benzoic acid dihydrochloride (**18**, 290 mg, 0.94 mmol) in CH₃CN (10 ml), DIPEA (0.8 ml, 4.7 mmol), EDC (215 mg, 1.13 mmol) and HOBt (165 mg, 1.22 mmol) were added and stirred for 10 minutes. 6-Methyl-N1-(4-(pyridin-3-yl)pyrimidin-2-yl)benzene-1,3-diamine (Formula III, 200 mg, 0.72 mmol) was added and the reaction mixture was stirred at room temperature for 12 h. After completion of the reaction, the reaction mixture was concentrated and the residue was purified on a Biotage Selekt flash purification system (silica gel, 10-15% MeOH/DCM) to afford *N*-(4-methyl-3-((4-(pyridin-3-yl)pyrimidin-2-yl)amino)phenyl)-4-((4-methyl-piperazin-1-yl)methyl)benzamide (Formula IV) as an off-white solid (292 mg, 82%).

¹H NMR (400 MHz, DMSO-*d₆*) δ ppm 10.16 (s, 1H), 9.28 (d J = 2.0 Hz, 1H), 8.97 (s, 1H), 8.69 (dd, J = 8.0, 2.0 Hz, 1H), 8.52 (d, J = 5.2 Hz, 1H), 8.49 (dt, J = 8.0, 2.0 Hz, 1H), 8.1 (d, J = 1.6 Hz, 1H), 7.92 (d, J = 8.0 Hz, 2H), 7.54 - 7.49 (m, 2H), 7.48 - 7.42 (m, 3H), 7.22 (d, J = 8.0 Hz, 1H), 3.54 (s, 2H), 2.47- 2.33 (m, 8H), 2.23 (s, 3H), 2.19 (s, 3H). ESI-MS *m*/*z* 492 [M-H]⁻. HPLC; Rt (min): 11.92 (Method 2).

### Synthesis of 3-(4-methyl-1H-imidazol-1-yl)-5-(trifluoromethyl)aniline (21)

3-Bromo-5-(trifluoromethyl)aniline (**19**, 4.8 g, 20 mmol), 4-methylimidazole (**20**, 1.97 g, 24 mmol), potassium carbonate (3.04 g. 22 mmol), Cul (0.57 g, 3 mmol) and 8-hydroxyquinoline (0.44 g, 3 mmol) were taken in dry DMSO (20 ml) in a pressure tube, and the suspension was purged under argon for 15 minutes. The tube was sealed and heated at 120 °C for 18 h. The reaction mixture was cooled to room temperature, and 10 ml of ammonium hydroxide solution (aq, 29%) was added and heated at 50 °C for 1 h. The reaction mixture was partitioned between EtOAc (30 ml) and H₂O (30 ml), and the aqueous layer was extracted with EtOAc (2 × 15 ml). The combined organic layer was dried (Na₂SO₄), concentrated and the residue was purified on a Biotage Selekt flash purification system (silica gel, 3-5% MeOH/DCM) to afford a pale green solid (3.6 g), which was recrystallised from EtOAc/Hexanes to afford 3-(4-methyl-1*H-*imidazol-1-yl)-5-(trifluoromethyl)aniline (**21**) as white needles (3.0 g, 63%). ¹H NMR (400 MHz, DMSO-*d₆*) δ ppm 8.07 (d, J = 1.2 Hz, 1H), 7.37 (s, 1H), 6.97 (s, 1H), 6.93 (s, 1H), 6.81 (s, 1H), 5.86 (s, 2H), 2.15 (s, 3H). ESI-MS *m*/*z* 242 [M + H]⁺. HPLC; Rt (min) : 6.98 (Method 1).

### Synthesis of 4-methyl-N-(3-(4-methyl-1H-imidazol-1-yi)-5-(trifluoromethyl)phenyl)-3-((4-(pyridin-3-yl)pyrimidin-2-yl)amino)benzamide (Nilotinib, Formula II)

### (a) From Formula I through the corresponding acid chloride

To a slurry of 4-methyl-3-((4-(pyridin-3-yl)pyrimidin-2-yl)amino)benzoic acid (Formula I, 306 mg, 1.0 mmol) in thionyl chloride (5 ml), a few drops of DMF were added and the reaction mixture was heated to reflux for 6 h. The reaction mixture was then concentrated *in vacuo* and the residue was dried under an argon atmosphere to afford the crude acid chloride, which was used without any further purification. The acid chloride was dissolved in CH₂Cl₂ (10 ml) and cooled to 0 °C . A solution of 3-(4-methyl-1*H*-imidazol-1-yl)-5-(trifluoromethyl)aniline (**21**, 242 mg, 1.0 mmol), Et₃N (0.42 ml, 3 mmol) and DMAP (5 mg) in CH₂Cl₂ (10 ml) was addded dropwise to the cold solution of acid chloride at 0 °C. The reaction mixture was stirred at room temperature for 6 h. The reaction mixture was diluted with CH₂Cl₂ (10 ml) and washed with a saturated NaHCOs solution (2 × 10 ml), brine solution (2 × 10 ml). The organic layer was dried (Na₂SO₄), concentrated and the residue was purified on a Biotage Selekt flash purification system (silica gel, 3-5 % MeOH/DCM), to afford 4-methyl-*N*-(3-(4-methyl-1*H*-imidazol-1-yl)-5-(trifluoromethyl)phenyl)-3-((4-(pyridin-3-yl)pyrimidin-2-yl)amino)benzamide as an off-white solid (Formula II) (334 mg, 63%).

### (b) From the corresponding methyl ester (6a)

To a mixture of methyl 4-methyl-3-((4-(pyridin-3-yl)pyrimidin-2-yl)amino)benzoate (**6a**, 500 mg, 1.56 mmol) and 3-(4-methyl-1*H*-imidazol-1-yl)-5-(trifluoromethyl)aniline (**21**, 376 mg, 1.56 mmol) kept under argon, THF (10 ml) was added and the solution was cooled to 0 °C in an ice bath. A solution of sodium *tert*-butoxide (1.05 g, 10.92 mmol) in THF (15 ml) was added dropwise. The reaction mixture was stirred at room temperature for 12 h. Brine solution (20 ml) was added to the reaction mixture and the reaction mixture was extracted with EtOAc (3 × 15 ml). The combined organic layers were dried (Na₂SO₄), concentrated and the residue was purified on a Biotage Selekt flash purification system (silica gel, 3-5 % MeOH/DCM), to afford 4-methyl-*N*-(3-(4-methyl-1*H*-imidazol-1-yl)-5-(trifluoromethyl)phenyl)-3-((4-(pyridin-3-yl)pyrimidin-2-yl)amino)benzamide as an off-white solid (670 mg, 81%).

¹H NMR (400 MHz, DMSO-*d₆*) δ ppm 10.61 (s, 1H), 9.29 (s, 1H), 9.16 (s, 1H), 8.69 (d, J = 4.8 Hz, 1H), 8.56 (d, J = 4.8 Hz, 1H) 8.46 (dt, J = 8.0, 2.0 Hz, 1H), 8.33 (d, J = 1.6 Hz, 1H), 8.30 (s, 1H), 8.21 (s, 1H), 8.17 (s, 1H), 7.78 (dd, J = 8.0, 2.0 Hz, 1H) 7.72 (s, 1H), 7.53-7.48 (m, 3H), 7.47 (d, J = 8.4 Hz, 1H), 2.37 (s, 3H), 2.18 (s, 3H). ESI-MS *m*/*z* 530 [M + H]⁺. HPLC; Rt (min): 8.49 (Method 1).

### References

A number of publications are cited above in order to more fully describe and disclose the invention and the state of the art to which the invention pertains.

## Claims

1. A method for the synthesis of a compound of Formula IIIa: 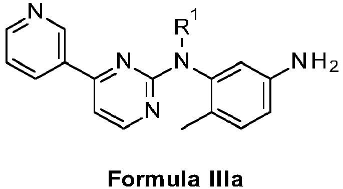
wherein R¹ is H or a nitrogen protecting group, the method comprising treating a compound of Formula Ia with reagents to form an acyl azide: 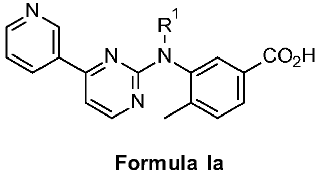
wherein R¹ is H or a nitrogen protecting group;
followed by decomposition of the acyl azide to provide an isocyanate; which isocyanate undergoes nucleophilic attack with an alcohol or water.

2. The method of claim 1, wherein the isocyanate undergoes nucleophilic attack with an alcohol R⁴OH to provide a compound of Formula XIII: 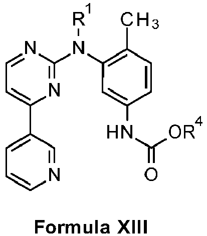 wherein R⁴ is an optionally substituted C₁₋₇ alkyl, optionally wherein R⁴ is selected from *t*-butyl and an optionally substituted benzyl group.

3. The method of claim 2, wherein R⁴ is selected from *t*-butyl, benzyl and 2,4-dimethoxybenzyl.

4. The method of any preceding claim, wherein the reagents for acyl azide formation comprise diphenylphosphoryl azide (DPPA).

5. The method of any preceding claim, wherein R¹ is *t*-butyloxycarbonyl.

6. The method of any preceding claim, wherein R¹ is not H and the method further comprises a deprotection step to remove the R¹ nitrogen protecting group;
optionally wherein the deprotection step uses trifluoroacetic acid and the deprotection affords a compound of Formula III as the free base: 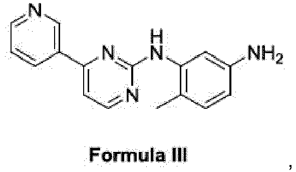
or optionally wherein the deprotection step uses hydrochloric acid and the deprotection affords a compound of Formula III as the hydrochloride salt: 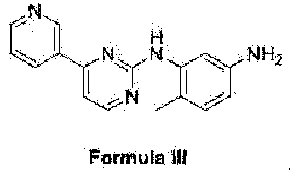 .HCl.

7. The method of any preceding claim, wherein the method further comprises the step of providing the compound of Formula la by effecting a coupling reaction between a compound of Formula XI: 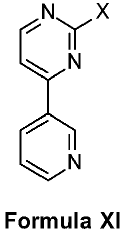
and a compound of Formula Xlla: 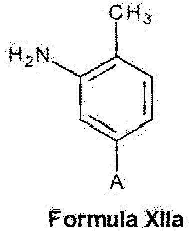
to provide a compound of Formula Villa: 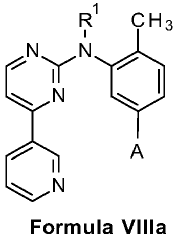
and hydrolysing the compound of Formula Villa to produce the compound of Formula Ia;
wherein R¹ is H;
X is a halogen, OTf or OTs, optionally CI;
and A is COOR² or CN;
wherein R² is an optionally substituted C₁₋₄ alkyl.

8. The method of claim 7, wherein the method further comprises providing the compound of Formula XI by coupling of a compound of Formula IX: 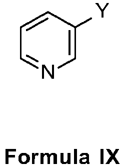
with a compound of Formula X: 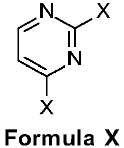
wherein each substituent X in Formula X is independently a halogen or OTf, optionally Cl;
and Y is a halogen, OTf, OTs, or other group suitable for cross coupling.

9. The method of claim 8, wherein Y is selected from a halogen, OTf, OTs, -SnR^{A}₃, -B(OH)₂ and 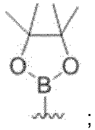 wherein R^{A} is C₁₋₄ alkyl.

10. The method of claim 9, wherein Y is a halogen, OTf or -B(OH)₂.

11. The method of any one of claims 8 to 10, the method comprising treating the compound of Formula IX with magnesium to form a Grignard reagent.

12. The method of any one of claims 8 to 10, the method comprising treating the compound of Formula IX with zinc to form an organozinc Negishi reagent.

13. The method of either claim 8 or claim 9, the method comprising providing a compound of Formula XI by Suzuki coupling of a compound of Formula IX with a compound of Formula X; wherein Y is -B(OH)₂ or

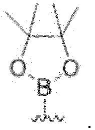

14. The method of any one of claims 1 to 6, wherein the method further comprises the step of providing the compound of Formula la by reacting a compound of Formula VII: 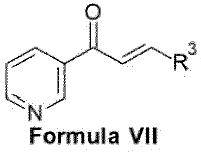
with a compound of Formula VIa: 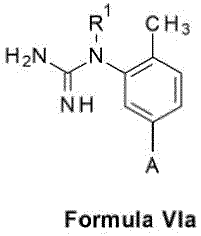
to provide a compound of Formula Villa: 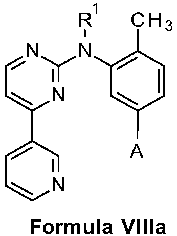
and hydrolysing the compound of Formula Villa to produce the compound of Formula la;
wherein Formula Vla is the free base or a chloride, bromide, iodide, mesylate, sulfate, phosphate, nitrate, acetate, oxalate, citrate, or tartrate salt; A is CN; R¹ is H or a nitrogen protecting group; and R³ is a suitable leaving group, optionally dialkyl-substituted nitrogen.

15. The method of claim 14, wherein R¹ is H and/or R³ is NMe₂.

## Patentansprüche

1. Verfahren zur Synthese einer Verbindung der Formel IIIa: 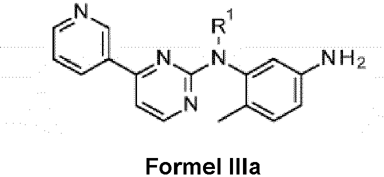
worin R¹ H oder eine Stickstoff-Schutzgruppe ist, wobei das Verfahren das Versetzen einer Verbindung der Formel Ia mit Reagenzien umfasst, um ein Acylazid zu bilden: 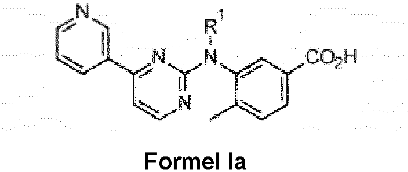
worin R¹ H oder eine Stickstoff-Schutzgruppe ist;
gefolgt von der Zersetzung des Acylazids, um ein Isocyanat bereitzustellen; wonach das Isocyanat einen nucleophilen Angriff mit einem Alkohol oder Wasser erfährt.

2. Verfahren nach Anspruch 1, wobei das Isocyanat einen nucleophilen Angriff mit einem Alkohol R⁴OH durchläuft, um eine Verbindung der Formel XIII bereitzustellen: 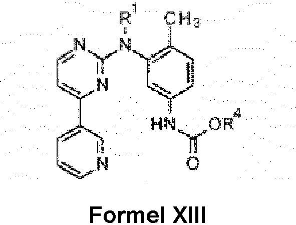 worin R⁴ ein gegebenenfalls substituiertes C₁₋₇-Alkyl ist, wobei R⁴ gegebenenfalls aus t-Butyl und einer gegebenenfalls substituierten Benzylgruppe ausgewählt ist.

3. Verfahren nach Anspruch 2, wobei R⁴ aus t-Butyl, Benzyl und 2,4-Dimethoxybenzyl ausgewählt ist.

4. Verfahren nach einem der vorangegangenen Ansprüche, wobei die Reagenzien zur Bildung des Acylazids Diphenylphosphorylazid (DPPA) umfassen.

5. Verfahren nach einem der vorangegangenen Ansprüche, wobei R¹ t-Butyloxycarbonyl ist.

6. Verfahren nach einem der vorangegangenen Ansprüche, wobei R¹ nicht H ist und das Verfahren weiters einen Entschützungsschritt zum Entfernen der Stickstoff-Schutzgruppe R¹ umfasst;
wobei im Entschützungsschritt gegebenenfalls Trifluoressigsäure eingesetzt wird und das Entfernen der Schutzgruppe eine Verbindung der Formel III als freie Base ergibt: 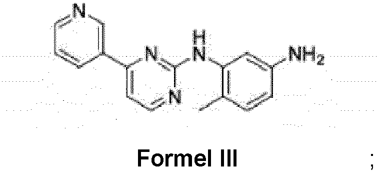
oder wobei im Entschützungsschritt gegebenenfalls Salzsäure eingesetzt wird und das Entfernen der Schutzgruppe eine Verbindung der Formel III als Hydrochlorid-Salz ergibt: 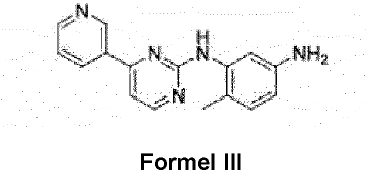 HCl.

7. Verfahren nach einem der vorangegangenen Ansprüche, wobei das Verfahren weiters den Schritt des Bereitstellens der Verbindung der Formel Ia mittels Durchführung einer Kupplungsreaktion zwischen einer Verbindung der Formel XI: 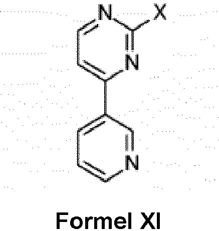
und einer Verbindung der Formel Xlla: 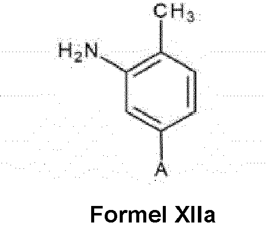
zum Bereitstellen einer Verbindung der Formel VIIIa: 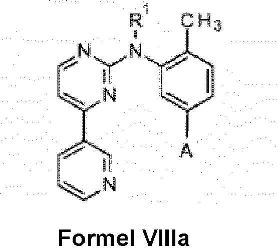
und das Hydrolysieren der Verbindung der Formel VIIIa umfasst, um die Verbindung der Formel la herzustellen;
worin R¹ H ist;
X ein Halogen, OTf oder OTs, gegebenenfalls Cl, ist;
und A COOR² oder CN ist;
worin R² ein gegebenenfalls substituiertes C₁₋₄-Alkyl ist.

8. Verfahren nach Anspruch 7, wobei das Verfahren weiters das Bereitstellen der Verbindung der Formel XI durch Kuppeln einer Verbindung der Formel IX: 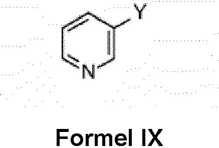
mit einer Verbindung der Formel X: 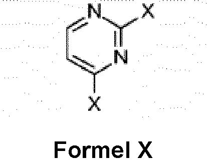
umfasst, wobei die Substituenten X in Formel X jeweils unabhängig ein Halogen oder OTf, gegebenenfalls Cl, sind; und Y ein Halogen, OTf, OTs oder eine andere Gruppe ist, die für eine Kreuzkupplung geeignet ist.

9. Verfahren nach Anspruch 8, wobei Y aus einem Halogen, OTf, OTs, -SnR^{A}₃, -B(OH)₂ und 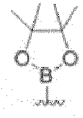 ausgewählt ist; worin R^{A} C₁₋₄-Alkyl ist.

10. Verfahren nach Anspruch 9, wobei Y ein Halogen, OTf oder -B(OH)₂ ist.

11. Verfahren nach einem der Ansprüche 8 bis 10 wobei das Verfahren das Versetzen der Verbindung der Formel IX mit Magnesium umfasst, um ein Grignard-Reagens zu bilden.

12. Verfahren nach einem der Ansprüche 8 bis 10, wobei das Verfahren das Versetzen der Verbindung der Formel IX mit Zink umfasst, um ein Organozink-Negishi-Reagens zu bilden.

13. Verfahren nach einem der Ansprüche 8 oder 9, wobei das Verfahren das Bereitstellen einer Verbindung der Formel XI durch Suzuki-Kupplung einer Verbindung der Formel IX mit einer Verbindung der Formel X umfasst; wobei Y -B(OH)₂ oder 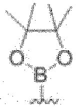 ist.

14. Verfahren nach einem der Ansprüche 1 bis 6, wobei das Verfahren weiters den Schritt des Bereitstellens der Verbindung der Formel Ia durch Umsetzen einer Verbindung der Formel VII: 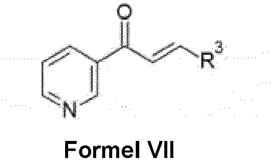
mit einer Verbindung der Formel Vla: 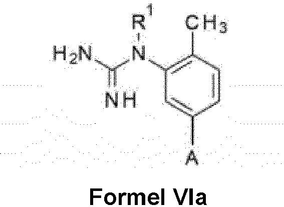
um eine Verbindung der Formel Villa bereitzustellen: 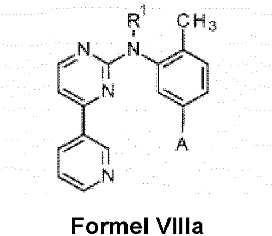
und das Hydrolysieren der Verbindung der Formel Villa umfasst, um die Verbindung der Formel Ia herzustellen; wobei Formel VIa die freie Base oder ein Chlorid-, Bromid-, lodid-, Mesylat-, Sulfat-, Phosphat-, Nitrat-, Acetat-, Oxalat-, Citrat- oder Tartrat-Salz ist; A CN ist; R¹ H oder eine Stickstoff-Schutzgruppe ist; und R³ eine geeignete Abgangsgruppe, gegebenenfalls Dialkyl-substituierter Stickstoff, ist.

15. Verfahren nach Anspruch 14, wobei R¹ H ist und/oder R³ NMe₂ ist.

## Revendications

1. Procédé de synthèse d'un composé de Formule IIIa : 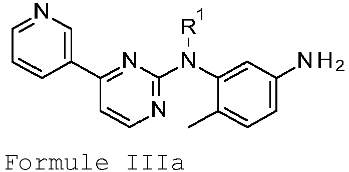
dans lequel R¹ est H ou un groupe protecteur d'azote, le procédé comprenant le traitement d'un composé de Formule Ia avec des réactifs pour former un azoture d'acyle : 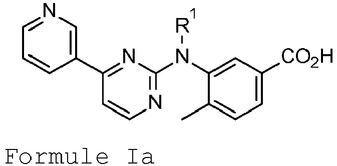
dans lequel R¹ est H ou un groupe protecteur d'azote ;
suivi de la décomposition de l'azoture d'acyle pour fournir un isocyanate ; lequel isocyanate subit une attaque nucléophile avec un alcool ou de l'eau.

2. Procédé selon la revendication 1, dans lequel l'isocyanate subit une attaque nucléophile avec un alcool R⁴OH pour fournir un composé de Formule XIII : 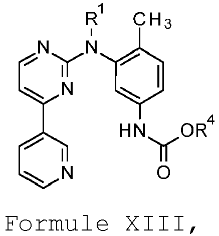 dans lequel R⁴ est un alkyle en C₁₋₇ facultativement substitué, facultativement dans lequel R⁴ est choisi parmi t-butyle et un groupe benzyle facultativement substitué.

3. Procédé selon la revendication 2, dans lequel R⁴ est choisi parmi *t*-butyle, benzyle et 2,4-diméthoxybenzyle.

4. Procédé selon l'une quelconque des revendications précédentes, dans lequel les réactifs pour la formation d'azoture d'acyle comprennent de l'azoture de diphénylphosphoryle (DPPA).

5. Procédé selon l'une quelconque des revendications précédentes, dans lequel R¹ est le t-butyloxycarbonyle.

6. Procédé selon l'une quelconque des revendications précédentes, dans lequel R¹ n'est pas H et le procédé comprend en outre une étape de suppression de protection pour éliminer le groupe protecteur d'azote R¹ ;
facultativement dans lequel l'étape de suppression de protection utilise de l'acide trifluoroacétique et la suppression de protection fournit un composé de Formule III comme base libre : 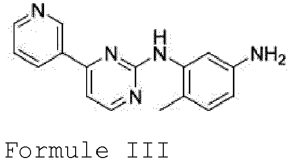
ou facultativement dans lequel l'étape de suppression de protection utilise de l'acide chlorhydrique et la suppression de protection fournit un composé de Formule III comme sel de chlorhydrate :

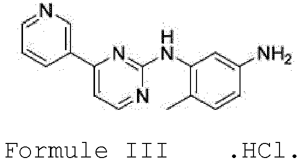

7. Procédé selon l'une quelconque des revendications précédentes, dans lequel le procédé comprend en outre l'étape consistant à fournir le composé de Formule Ia en effectuant une réaction de couplage entre un composé de Formule XI : 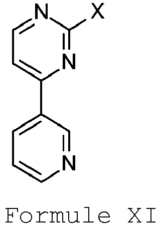
et un composé de Formule XIIa : 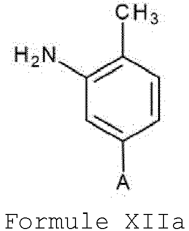
pour fournir un composé de Formule VIIIa : 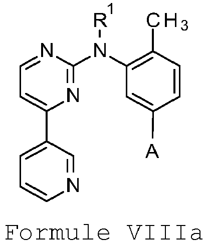
et à hydrolyser le composé de Formule VIIIa pour produire le composé de Formule Ia ;
dans lequel R¹ est H ;
X est un halogène, OTf ou OTs, facultativement Cl ;
et A est COOR² ou CN ;
dans lequel R² est un groupe alkyle en C₁-₄ facultativement substitué.

8. Procédé selon la revendication 7, dans lequel le procédé comprend en outre la fourniture du composé de Formule XI par couplage d'un composé de Formule IX : 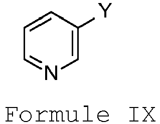
avec un composé de Formule X : 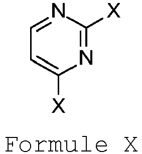
dans lequel chaque substituant X dans la Formule X est indépendamment un halogène ou OTf, facultativement Cl ;
et Y est un halogène, OTf, OTs, ou un autre groupe approprié pour un couplage croisé.

9. Procédé selon la revendication 8, dans lequel Y est choisi parmi un halogène, OTf, OTs, -SnR^{A}₃, -B(OH)₂ et 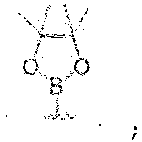 dans lequel R^{A} est alkyle en C₁-₄.

10. Procédé selon la revendication 9, dans lequel Y est un halogène, OTf ou -B(OH)₂.

11. Procédé selon l'une quelconque des revendications 8 à 10, le procédé comprenant le traitement du composé de Formule IX avec du magnésium pour former un réactif de Grignard.

12. Procédé selon l'une quelconque des revendications 8 à 10, le procédé comprenant le traitement du composé de Formule IX avec du zinc pour former un réactif organozinc de Negishi.

13. Procédé selon la revendication 8 ou la revendication 9, le procédé comprenant la fourniture d'un composé de Formule XI par couplage de Suzuki d'un composé de Formule IX avec un composé de Formule X ; dans lequel Y est -B(OH)₂ ou

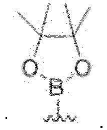

14. Procédé selon l'une quelconque des revendications 1 à 6, dans lequel le procédé comprend en outre l'étape consistant à fournir le composé de Formule Ia en faisant réagir un composé de Formule VII : 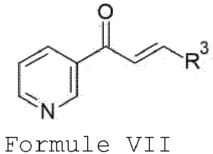
avec un composé de Formule VIa : 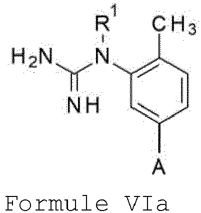
pour fournir un composé de Formule VIIIa : 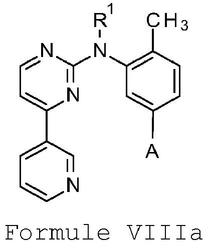
et à hydrolyser le composé de Formule VIIIa pour produire le composé de Formule Ia ;
dans lequel la Formule VIa est la base libre ou un sel de chlorure, de bromure, d'iodure, de mésylate, de sulfate, de phosphate, de nitrate, d'acétate, d'oxalate, de citrate ou de tartrate ; A est CN ; R¹ est H ou un groupe protecteur d'azote ; et R³ est un groupe labile approprié, facultativement de l'azote substitué par dialkyle.

15. Procédé selon la revendication 14, dans lequel R¹ est H et/ou R³ est NMe₂.
